(19) Europäisches Patentamt
European Patent Office
Office européen des brevets

(11) **EP 4 350 704 A1**

(12) **EUROPEAN PATENT APPLICATION**

(43) Date of publication:
**10.04.2024 Bulletin 2024/15**

(21) Application number: **22199421.3**

(22) Date of filing: **03.10.2022**

(51) International Patent Classification (IPC):
**G16H 30/40** (2018.01)   **G16H 50/70** (2018.01)
**G06N 3/045** (2023.01)

(52) Cooperative Patent Classification (CPC):
**G16H 50/70; G06N 3/045; G16H 30/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **University of Leeds**
**Leeds LS2 9JT (GB)**

(72) Inventors:
• **DOU, Haoran**
  **Leeds, LS2 9BW (GB)**
• **RAVIKUMAR, Nishant**
  **Leeds, LS2 9BW (GB)**
• **FRANGI, Alejandro**
  **LS2 9BW, Leeds (GB)**

(74) Representative: **HGF**
**HGF Limited**
**1 City Walk**
**Leeds LS11 9DX (GB)**

(54)   **METHOD AND APPARATUS FOR GENERATING VIRTUAL POPULATIONS OF ANATOMY**

(57)   There is provided a computer-implemented method for generating virtual chimera populations of multi-part organ shapes for use in an in-silico trial, wherein organ shapes are represented as surface or volumetric meshes, the method comprising using a part-aware generative model to learn a latent representation of each part of a multi-part organ shape for inclusion in a virtual population and output synthesised parts of the multi-part organ shape, using a spatial composition model to align the outputted synthesised parts of the multi-part organ shape and output an anatomically meaningful example of an overall multi-part organ shape as a virtual chimera and storing the virtual chimera in the virtual population, for use in the in-silico study. There is also provided an apparatus and computer readable medium embodying the method.

Figure 1

**Description**

**FIELD OF THE INVENTION**

The present invention relates to a computer-implemented method, computer program and apparatus for generating virtual populations of anatomy for use in in-silico studies, and the use thereof for medical device or drug design, manufacture, testing and regulatory approval.

**BACKGROUND OF THE INVENTION**

The development costs of medical devices have grown steadily in recent years, driven by lengthy regulatory approval processes and costly clinical trials. The high rate of late-phase failures of medical devices in clinical trials can lead to significant financial losses for medical device manufacturers, and the financial losses incurred by such late-phase failures are often subsequently rolled over onto the much smaller number of successful medical devices that are eventually approved. The resulting financial burden on healthcare systems and the medical device industry worldwide is fast becoming unsustainable, and there is a clear need for a paradigm shift in medical device innovation and regulatory approval processes.

Traditional (i.e. in-vivo) clinical trials for medical devices are often restricted by the limited variability in operational regimes (e.g. patient variability) that medical devices are able to be subjected to when evaluating their safety and efficacy. This may be due to the difficulty of recruiting sufficient patients that cover the variability of target populations for the medical device under development (i.e. the target patient as intended for use with the developed medical devices in routine clinical care). Insufficient validation of medical device safety and efficacy can lead to serious adverse side effects only becoming apparent post-market approval, particularly when used in operational regimes not assessed adequately in pre-market approval clinical trials.

However, in-silico trials (ISTs) provide an alternative and complementary route to medical device innovation, and the regulatory approval process, which underpins the route to commercialisation of medical devices and their adoption in routine patient care. ISTs use computational modelling and simulation to evaluate the safety and efficacy of medical devices virtually, in virtual patient populations and provide digital (or in-silico) as opposed to real-world evidence in support of the regulatory approval of the medical devices under development. ISTs thus have the potential to explore medical device performance in a wider range of patient characteristics than would be feasible to recruit for in a real clinical trial, and hence ISTs can help refine, reduce, and partially replace in-vivo clinical trials for medical device testing.

**SUMMARY**

The technology described herein provides a generative framework that enables simulation of animal, including human, anatomy and physiology, for medical device or drug design and testing. In particular, synthesis of plausible multi-part organs (or multi-organ shape assemblies) using shapes of macroscopic anatomical structures represented as unstructured graphs as input data. The disclosed generative framework can model each part/organ individually, or jointly, and then encode single or multi-part anatomical shapes into a latent representation (i.e. low-dimensional representation, for example a vector) that may be decoded to synthesise instances of each part individually, or of multiple parts simultaneously. The synthesised parts may be spatially composed (i.e. combined together, spatially) to generate plausible multi-part/multi-organ shape assemblies referred to herein as 'virtual chimeras'. Advantageously, the described methods, and associated apparatuses, allow non-/partially-overlapping information from different datasets to be used for training, and then subsequently, synthesising virtual chimera populations using the trained system. For example, in the specific area of Cardiac modelling, aortic vessel geometries extracted from computed tomography angiography (CTA) data of a clinical trial cohort may be combined with cardiac chamber geometries extracted from cine-magnetic resonance images (cine-MRI) acquired across a general population, such as may be provided by the UK Biobank, to produce complete cardiac virtual chimera populations.

Generating cardiac virtual populations that include aortic vessel geometries is not viable using cardiac cine-magnetic resonance images alone, because the field of view of that sort of image does not capture the aorta. However, cardiac CTA images do capture the aorta, and enable their geometries to be extracted and characterised in 3D. A benefit of the proposed approach is that it enables parts of organs/organs extracted from one type of image (i.e. imaging modality) for a specific population (e.g. aortas from CTA of a Transcatheter aortic valve implantation (TAVI) clinical trial cohort) to be combined with other parts of the same organ/other organs from one or several other imaging modalities, which may even be acquired for different patient populations (e.g. cardiac chambers from cine-magnetic resonance imaging of a population imaging initiative such as the UK Biobank).

The disclosed method may also comprise a composition neural network to spatially compose real or synthesised anatomical parts belonging to a multi-part or multi-organ assembly of interest. In such examples, the composition neural network may learn the spatial relationships between adjacent anatomical parts and/or organs of interest and recover affine transformation (i.e. linear mapping) and non-rigid spatial transformations that are able to configure the parts and/or organs into anatomically plausible combined (overall) assemblies. Beneficially, such a composition neural network may also be independent of the availability of completely overlapping data for training, for example because it is built through self-supervision and hence can be trained with both non-overlapping and partially-overlapping data.

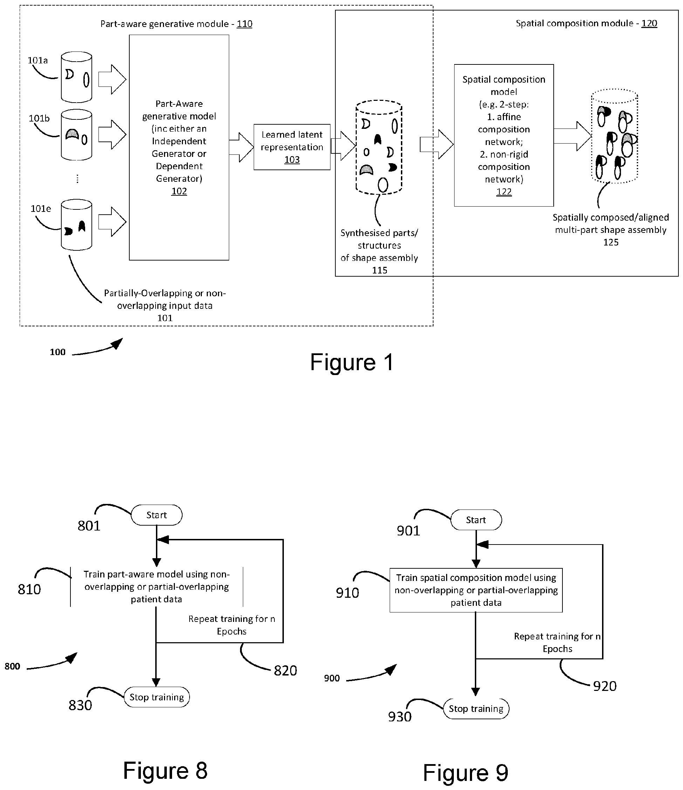

Compared with prior known technologies that are dependent on the availability of completely overlapping data in the training population, i.e. they require all parts and or organs structures to be available for each patient included in the training population, the disclosed approach(es) can generate more diverse virtual patient populations, and can exploit cross-modality and cross-population data sources. The disclosed generative framework for synthesising virtual chimera populations is described by the schematic shown in Figure 1, in the specific use-case of generating multi-part whole heart (cardiac). However, the disclosed approach(s) can be similarly used to synthesise any multi-part or multi-organ shape assemblies (for example, blood vessels, bone structures, or any other sub-selection of a physical entity within an anatomy).

An advantage of the present disclosure is to enable disparate medical datasets, with potentially only non-, or only partially-overlapping anatomical structures to be leveraged for learning a generative model of multi-part anatomical shape assemblies. As such, examples of the present disclosure tackle the problem of creating models (i.e. so-called 'virtual chimeras') by combining data from different subjects (or datasets) with partially-overlapping (or non-overlapping) anatomical structures, in a generative shape modelling framework that is capable of synthesising multi-part shape assemblies that are representative of realistic native anatomy.

The disclosed approaches to generative shape compositional learning utilise composition networks that predict rigid or affine transformations to compose the individual parts synthesised into coherent multi-part assemblies, however these may be extended to include a non-rigid registration component in the composition neural network to 'stitch' the individual parts (anatomical structures) together.

Advantages of the present disclosure may include: the ability to use disparate patient datasets for training the different parts of the disclosed generative framework, for example those with non-overlapping and with partially-overlapping anatomical structures/multi-part shape assemblies; providing a generative framework that can effectively capture the variability in shape of each individual part in an organ or organ assembly, and that is able to accommodate varying topology across all parts within the organ or organ assembly. Whilst remaining anatomically realistic, and finally, is not dependent on the availability of all parts of the shape assembly, across all samples in the training set (i.e. does not require fully-overlapping input data).

## BRIEF DESCRIPTION OF THE DRAWINGS

Examples of the invention are further described hereinafter with reference to the accompanying drawings, in which:

Figure 1 shows an example system for generating virtual organ assemblies (or training thereof) according to an example of the disclosure;
Figure 2 shows an example independent generator for use in the system of Figure 1, according to an example of the disclosure;
Figure 3 shows an example dependent generator for use in the system of Figure 1, according to an example of the disclosure;
Figure 4 shows an example architecture of a residual graph convolutional down-sampling block for use in all graph neural networks in the system of Figure 1, according to an example of the disclosure;
Figure 5 shows an example architecture of a residual graph convolutional up-sampling block for use in all graph neural networks in the system of Figure 1, according to an example of the disclosure;
Figure 6 shows an example graph neural network based architecture for an affine spatial composition network for use in the spatial composition module of Figure 1, according to an example of the disclosure;
Figure 7 shows an example graph neural network based architecture for a non-rigid spatial composition network for use in the spatial composition module of Figure 1, according to an example of the disclosure;
Figure 8 shows a method of training a part-aware model, according to an example of the disclosure;
Figure 9 shows a method of training a spatial composition model, according to an example of the disclosure;
Figure 10 shows a method of generating a virtual population for use in in-silico trials, according to an example of the disclosure;
Figure 11 shows an apparatus for carrying out the method of generating a virtual population for use in in-silico trials, according to an example of the disclosure.

## DETAILED DESCRIPTION

The technical operation and advantages of the present disclosure shall now be provided by way of a plurality of examples that are merely illustrative of the novel and inventive features, and the disclosed examples are intended to be fully combinable in any reasonable combination, unless explicitly stated otherwise (for example, are defined as true alternatives).

Figure 1 shows an example system 100 for generating virtual organ assemblies according to an example of the disclosure, in general terms. The disclosed system 100, in its most generic form (also referred to as a generative shape modelling system), comprises two main components - a part-aware generative module 110 and a spatial composition module 120. The part-aware generative module 110 comprises a part-aware generative model block 102, which takes as input, one or more sets of patient data 101 (e.g. partially-overlapping data, or non-overlapping data, in the form of meshes 101a-101e), and provides as output a learned latent representation of those meshes 103, which in turn are used to define a set of synthesised parts (of the organ), or (sub-)structures of the organ shapes (in, for example, multi-organ assemblies) 115. The spatial composition module 120 comprises a spatial composition model block 122, which takes as input the synthesised parts/structures 115, and spatially aligns them to form a spatially composed, realistic set of parts (e.g. organ parts) of a multi-shape part, or assembly of parts 125 (e.g. of multi-part organ assemblies).

According to an example, both modules 110 and 120 are parameterised (defined by) graph convolutional neural networks. A generative model, as used in the part-aware generator model block 102, is an algorithm that is able to learn from real data and synthesise virtual instances that are similar to, but not the same as, the real data instances that it is trained with. The part-aware generative model 102 is used for synthesising one or more virtual instances of anatomical parts/shapes of a multi-part organ (i.e. a single organ, having a predefined set of parts, or sub-portions), or multi-organ shape or assembly (i.e. multiple organs, comprising a predefined set of parts/sub-portions). In the following example disclosure, the example used will be a human heart comprising multiple parts/structures. However, other undescribed examples may include multiple abdominal organs such as liver, kidneys, pancreas, etc.). The spatial composition model 122 is used to spatially organise (i.e. move/align, or 'compose') the one or more synthesised virtual instances of the parts (of the organ, or organ assembly) into anatomically meaningful multi-part/multi-organ shape assemblies. The individual spatially composed multi-part/multi-organ shape assemblies output by the spatial composition model 122 are referred to as 'virtual chimeras' (which may be thought of as a singular complete model of a respective organ, or organ assembly), and may be combined together resulting in a 'virtual population' created by generating multiple such instances of virtual chimeras, which may be referred to as a 'virtual chimera population'.

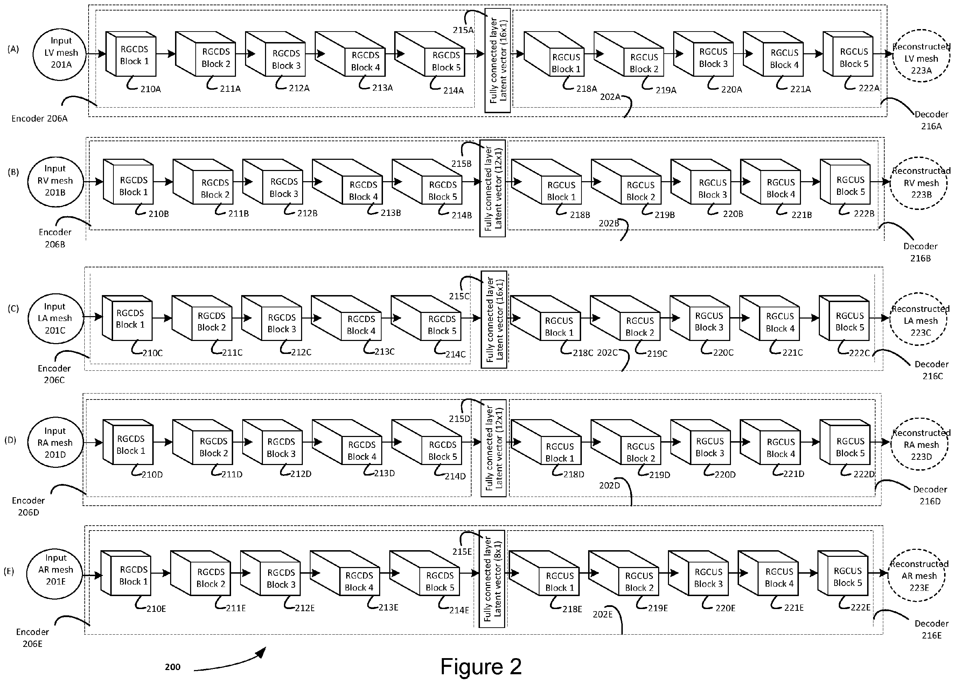

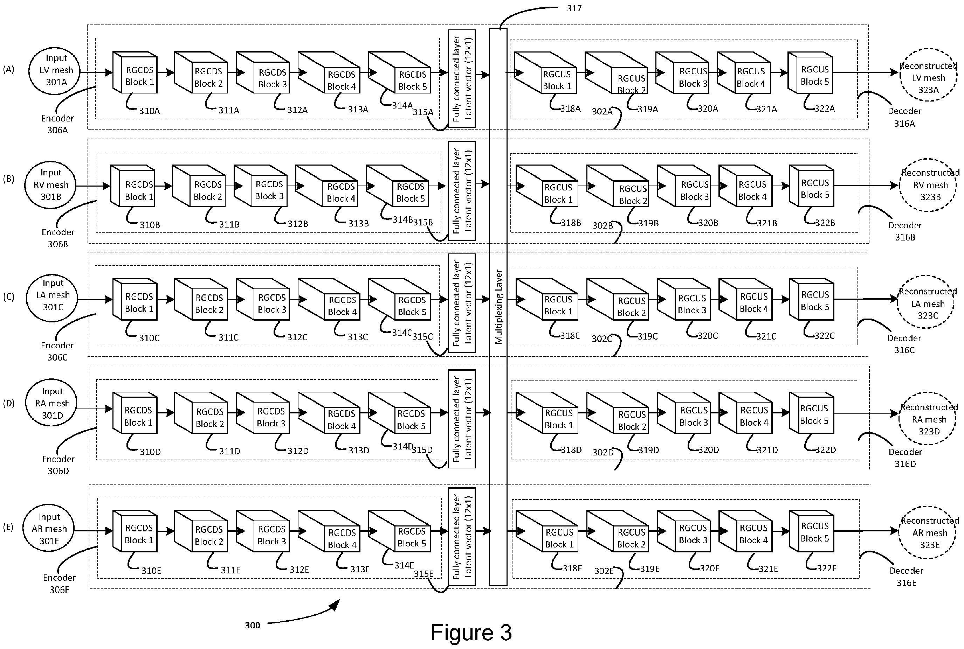

As noted above, Figure 1 depicts a schematic diagram that provides an overview of the generative shape modelling system 100 for synthesising virtual chimera populations from non-overlapping and partially-overlapping data sets of anatomical shapes. In this example, the partially/non-overlapping nature of the input data used is shown by the shading. The first step/component of the generative shape modelling system 100 is a part-aware generative model 102, where the part-aware generative model has two distinct implementations - an independent generator implementation and a dependent generator implementation. The two different implementations are not used together - either the independent generator is used, or the dependent generator used, i.e. they are two ways of tackling the same problem. The independent generator implementation is depicted by the schematic shown in Figure 2, which illustrates how the independent generator can be constructed and trained to learn individual latent representations (i.e. hidden low-dimensional representations) for each anatomical part or organ of interest independently from the others. Meanwhile, the dependent generator implementation is shown in Figure 3, and is constructed and trained to learn a shared latent representation across multiple parts' and/or multiple organs' shapes of interest jointly, in order to synthesise the desired multi-part/multi-organ shape assemblies.

In an example based on cardiac modelling, the generative shape modelling system 100 can be used to learn individual or shared latent representations for anatomical parts that constitute a human heart, which in the present example comprises the left ventricle (LV), right ventricle (RV), left atrium (LA), right atrium (LA) and the aortic root (AR), using data from example patient data covering different individuals (and/or coming from different data sources), and particularly those lacking complete overlap of the example patient data - i.e. where all anatomical parts are not available for all patients' data used to build the system. The disclosed generative shape modelling system 100 is not restricted to modelling the abovementioned cardiac structures but may be extended to include additional cardiac structures if available, such as, coronary arteries and their branches, pulmonary artery, pulmonary veins, cardiac valves, etc. Additionally, the disclosed system 100 is not restricted to cardiac modelling and can be used to model other organs as multi-part shape assemblies (e.g. lungs, liver, spine) or even multiple organs as instances of a multi-part shape assembly (e.g. thoracic organs and abdominal organs). This is to say, whilst the specific exemplary embodiments described herein focus on generating cardiac virtual cohorts, other embodiments may be used to synthesise virtual cohorts of other multi-part organs or multi-organ shape assemblies.

Referring now to Figure 2, the independent generator 200 may comprise multiple (N) independent generator sub-models parameterised by (i.e. defined by) graph convolutional variational autoencoders (gcVAEs) - 202(A) to 202(E), each sub-model (i.e. gcVAE) corresponding to one of each of the N anatomical parts and/or organs that are to be synthesised to create the multi-part/multi-organ shape assemblies of interest. In the example of Figure 2, N=5 since there is a gcVAE provided for each of: the left ventricle, right ventricle, left atrium, right atrium, and aortic root, which combine to constitute the multi-part shape assembly (which, in this case, is the human heart). This is to say, sub-model (A) is a part-specific gcVAE network architecture used in the independent generator 200 to learn variability in left ventricular (LV) shapes observed across the training population; sub-model (B) is a part-specific gcVAE network architecture used to learn variability in right ventricular (RV) shapes observed across the training population; sub-model (C) is a part-specific gcVAE network architecture used to learn variability in left atrial (LA) shapes observed across the training population; sub-model (D) is a part-specific gcVAE network architecture used to learn variability in right atrial (RA) shapes observed across the training population; and sub-model (E) is a part-specific gcVAE network architecture used to learn variability in aortic root (AR) shapes observed across the training population.

Once trained, each part specific gcVAE 202(A)-202(E) can be sampled from, to generate new parts (of organs/organ assemblies) that are representative of the input training data set, but are not exactly the same as any input data set used in the training - i.e. they can now generate synthetic examples that are known to be realistic.

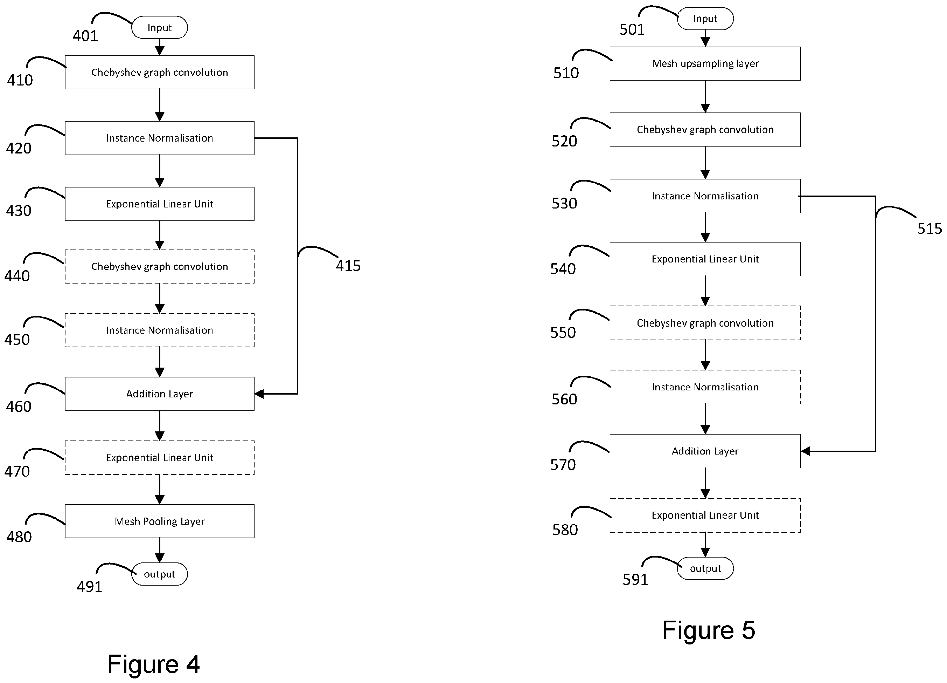

Each independent generator 200 comprises a set of graph-convolutional variational autoencoders (gcVAEs) 202A-202E. Each gcVAE 202 comprises a pair of neural networks referred to as the encoder (e.g. encoder 206A) and decoder (e.g. decoder 216A), where the encoder comprises a plurality of residual graph convolution down-sampling (RGCDS) blocks (e.g. 210A-214A), and the decoder comprises a plurality of residual graph convolution up-sampling (RGCUS) blocks (e.g. 218A-222A). The RGCDS and RGCUS blocks are discussed in more detail with respect to Figures 4 and 5 below. As shown in Figure 2, each of the 5 sub-models (denoted by the letters A-E) takes as input a mesh for the respective part (e.g. LV mesh 201A for the sub-model gcVAE 202 (A), etc.), which is down-sampled in the encoder 206, to form a fully connected layer Latent vector 215. Each of the five different sub-models can have a different vector size applied, and in the example shown, these are 16x1 for the LV and LA, 12x1 for the RV and RA, and 8x1 for the AR. The size of the latent vectors for each of the five different sub-models are determined empirically and can take on different sizes, for different applications and datasets used for training. This latent vector is then up-sampled in the decoder 216, to form an output reconstructed mesh 223 - e.g. reconstructed LV mesh 223A.

In more detail: each gcVAE is trained separately to learn individual and independent latent representations for each of the N corresponding anatomical parts and/or organs of interest. Variational autoencoders (VAEs) (and the graph-convolution variants, i.e. gcVAEs, used in the independent generator 200 disclosed herein) may be Bayesian latent variable models that couple 1) a recognition function represented by the encoder 206 network, to infer a low dimensional hidden representation of the input data (i.e. to infer a latent representation), with 2) a generative function represented by the decoder 216 network, which transforms the inferred latent representation back to the original data space. VAEs infer the latent representation of input data by approximating the posterior distribution of the latent variables given the observed input data. This is achieved by jointly optimising the encoder 206 and decoder 216 networks, for example to maximise the associated evidence lower bound (ELBO) of the observed input data. VAEs are flexible and can be used to learn latent representations of any type of data (e.g. images, audio signals, mesh-based representations of shapes). In the independent generator 200 of the example generative shape modelling system 100, VAEs are combined with graph-convolutional neural networks to learn latent representations of anatomical shapes represented by computational meshes (also referred to as unstructured graphs).

The encoder-decoder neural network pairs in both the independent 200 and dependent 300 generator (i.e. 206A/216A - 206E/216E, and 306A/316A - 306E/306E) are graph-convolutional neural networks that utilise Chebyshev convolution operations to extract a hierarchy (i.e. across multiple scales) of shape features and learn a representative latent representation describing variability in shape of the corresponding part(s) and/or organ(s) of interest across the training population. Each level has its own set of up and down sampling blocks (e.g. 210A/218A), thus, in the examples shown, there are 5 levels used, but other numbers of levels may be used instead. Also, the same number of levels are used across all example Figures, but different numbers of levels may be used in the different parts. The number of levels typically affects processing speed and accuracy, so a value that is the best compromise for a given use-case may be chosen for each implementation. The number of levels used in the developed independent and dependent generator were determined empirically to be the best for the data used to train the developed system. Given different training data for the same or different applications, the chosen number of levels may vary. Spatial convolution operations used in convolution neural networks are well-defined for structured/gridded data in the Euclidean domain and cannot be applied directly to irregularly structured data such as graphs. Chebyshev convolution operations provide a generalisation of spatial convolutions, enabling the spatial convolutions to be applied to data defined by graphs (i.e. meshes). Chebyshev convolution operations are performed in the Fourier domain. According to the convolution theorem, a convolution operation performed in the spatial domain is equivalent to element-wise multiplication in the Fourier domain. Chebyshev convolutions are applied by first transforming the graph-based data to the Fourier domain. The Fourier transformed graph-based data is then multiplied elementwise with the Fourier transformed convolution filter, and the inverse Fourier transform is subsequently applied to convert the result of the multiplicative operation back to the spatial domain. The parameters/weights of the convolution filter in a Chebyshev convolution operation are parameterised by truncated Chebyshev polynomials, where the polynomial coefficients represent the learnable weights/parameters of the convolution filter. Truncated Chebyshev polynomials are used in the disclosed generative shape modelling system as they reduce the computational complexity relative to other spectral convolution operations and relative to using general N-dimensional Chebyshev polynomials. However, other spectral convolution operations may also be used instead of truncated Chebyshev polynomial convolution operations, according to examples. Alternatively, spatial convolution operations designed for graph-based data, such as Feature-steered graph convolution operations may also be used instead of spectral/Chebyshev convolution operations.

The example independent generator 200, as shown in Figure 2, comprises five part-specific gcVAEs corresponding to the five cardiac structures of interest. To train each of the N=5 gcVAEs, consider an input data set denoted $X^{n=1...N} = \{x_i\}_{i=1...D}$ where, each $x_i$ denotes the shape of an anatomical part (e.g. left ventricle) of an individual (i.e. patient) represented in the training data set as a computational mesh (also known as a triangular surface mesh, or an unstructured graph). Each computational mesh is represented by a list of 3D points (i.e. spatial coordinates, which may be referred to as vertices or nodes) defining the boundary of the anatomical part/shape, and an adjacency matrix defining vertex/node connectivity. Each individual's data in the input data set $X^n$ is denoted by subscript $i$, where $\{i=1...D\}$ and D individuals' data is included in $X^n$. Each $X^n$ is the input data used to train each of the $\{n=1...N\}$ part-specific gcVAEs, and so in this example N=5. The D individuals whose data constitute each $X^n$ do not have to be the same, i.e. the training data set samples $\{x_i\}_{i=1...D}$ in each $X^n$ may come from the same or different individuals. In other words, the data sets $X^n$ used to train each gcVAE can comprise non-overlapping, partially-overlapping or completely overlapping data, where non-overlapping data means that all samples in each $X^n$ are obtained from different individuals; partially-overlapping data means that some proportion of samples in each $X^n$ are obtained from the same individuals; and completely overlapping data means that all samples in each $X^n$ are obtained from the same individuals. Each encoder network in each gcVAE takes a mesh -based representation of the shape for a given anatomical part or organ as input i.e. $x_i$ and maps this high-dimensional representation of shape to a low-dimensional vector or latent representation (denoted $z_i$). The low-dimensional vector or latent representation resulting from each sample being passed through the encoder 206 network, is subsequently mapped back or reconstructed to approximate the original mesh of the input shape, by the decoder 216 network. By training each gcVAE 202A-202E on its corresponding data set $X^n$, each latent representation for each data set $X^n$ is discovered separately (i.e. independently to the discovery for the other parts).

Each gcVAE 202A-202E is trained independently, using its corresponding input training data set $X^n$, to learn a latent representation from which the observed data $X^n$ is assumed to be generated, by approximating the true but intractable posterior distribution of the latent variables using variational inference. Variational inference is achieved by optimising the evidence lower bound (ELBO) of the observed data $X^n$ with respect to the parameters of the encoder-decoder networks in each gcVAE, where the encoder approximates the posterior distribution (denoted $q(z^n \mid X^n)$) given the input data $X^n$, and the decoder approximates the data likelihood given the latent variables (denoted $p(X^n \mid z^n)$). Thus, during training, for a given input sample $x_i$ the encoder maps the input to a unique latent representation $z^n_i$ which in turn is used as input by the decoder to reconstruct the data (denoted $r_i$). Put another way, during training the encoder learns to reduce the inputted data to a low-dimensional or compressed form, and the decoder learns to unravel or transform/map this compressed information back to an approximation of the data inputted to the encoder. Once the encoder and decoder are trained, in the generation phase (also referred to as inference phase or synthesis phase) new compressed forms of the data may be created and transformed by the trained decoder to generate synthetic data that appear realistic (i.e. has certain properties or characteristics that are shared and/or similar to real data).

Each gcVAE 202A-202E is trained independently, by jointly optimising their constituent encoder 206 and decoder 216 networks to maximise the associated evidence lower bound (ELBO) of the observed input data. The ELBO is formulated as a loss function comprising a summation of the expected negative log-likelihood of the data (denoted $L_{recon}$), the Kullback-Leibler divergence of the approximate posterior distribution of the latent variables $q(z^n \mid X^n)$ from the assumed prior distribution $p(z^n)$ over the latent variables (denoted $L_{KL}$), and an additional regularisation loss (denoted $L_{regular}$). The overall loss function ($L_{total}$) used to train each part-specific gcVAE 202A-E in the independent generator 200 is given by:

$$L_{total} = L_{recon} + w_0 L_{KL} + L_{regular} \quad (1)$$

where, $L_{recon}$ is a reconstruction loss used to represent the negative log-likelihood of the data, which is computed as the vertex-wise $L_1$- norm evaluated between each reconstructed ($r_i$) and original input shape/mesh ($x_i$). $L_{recon}$ is formulated as:

$$L_{recon} = ||x_i - r_i||_1 \quad (2)$$

The Kullback-Leibler divergence loss term $L_{KL}$ is a distance measure between probability distributions and here it is used to minimise the divergence between the approximated posterior distribution over the latent variables $q(\mathbf{z}^n \mid \mathbf{X}^n)$ and the true unknown (or target) posterior distribution over the latent variables $p(\mathbf{z}^n \mid \mathbf{X}^n)$. However, as the target posterior distribution $p(\mathbf{z}^n \mid \mathbf{X}^n)$ is intractable (cannot be estimated) in the present setting, it is factorised (expressed as) as a product of the data likelihood $p(\mathbf{X}^n \mid \mathbf{z}^n)$ and the assumed prior distribution over the latent variables $p(z)$ using Bayes' theorem, As a result the Kullback-Leibler divergence loss term $L_{KL}$ reduces to minimising the divergence between the approximated posterior distribution over the latent variables $q(\mathbf{z}^n \mid \mathbf{X}^n)$ and the prior distribution over the latent variables $p(\mathbf{z}^n)$. The prior distribution over the latent variables is assumed to be a centered isotropic multivariate Gaussian distribution, i.e. $p(\mathbf{z}) = \mathcal{N}(\mathbf{0}, \mathbf{I})$ for all n={1... N} part-specific GcVAEs. $w_0$ denotes the weight that is multiplied with the $L_{KL}$ term in order to balance its influence on the training process relative to the other terms in the overall loss function. And, the additional regularisation loss term $L_{regular}$ penalises outliers and encourages reconstructed outputs (i.e. reconstructed shapes on anatomical parts input to the encoder) from the decoder ($\mathbf{r}_i$) to be smooth. The regularisation loss $L_{regular}$ is formulated as a weighted summation of the Laplacian smoothness loss and an edge length loss. The Laplacian loss, denoted $L_{laplacian}$, encourages vertices within a neighbourhood to move together, i.e. it penalises neighbouring vertices in the reconstructed shape/mesh from moving apart from one another. Similarly, the edge length loss, denoted $L_{edge}$, penalises spurious motion of vertices relative to its neighbouring vertices. The regularisation loss $L_{regular}$ is given by:

$$L_{regular} = w_1 L_{laplacian} + w_2 L_{edge} \quad (3)$$

Here, $w_1$ and $w_2$ denote the weights that are multiplied with the $L_{laplacian}$ and $L_{edge}$ term, respectively, in order to balance their relative influence on the regularisation loss term and the overall training process, relative to the other terms in the overall loss function ($L_{total}$). $L_{laplacian}$ is given by:

$$L_{laplacian} = \sum_v \left( v - \sum_{v_k \in N(v)} \frac{v_k}{||N(v)||} \right) \quad (4)$$

where, v denotes each vertex/node in each input shape/mesh ($\mathbf{x}_i$) and $\mathbf{v}_k \in N(\mathbf{v})$ denotes its neighbouring vertices. And, $L_{edge}$ is given by:

$$L_{edge} = \sum_v \sum_{v_k \in N(v)} ||v - v_k||_2 \quad (5)$$

Each of the N=5 gcVAEs 202A-E in the independent generator 200, corresponding to the five cardiac structures of interest (LV, RV, LA, RA, AR), may be trained independently of each other by minimising the overall loss function $L_{total}$ with respect to their constituent encoder 206 and decoder 216 networks' parameters respectively. The parameters of the encoder-decoder network pairs in each gcVAE 202 are learned iteratively, for example via the error backpropagation algorithm.

There now follows an overview of an example specific network architecture that may be used for the part-specific gcVAEs in the independent generator 200 as depicted in Figure 2. The encoder-decoder network pairs in each gcVAE 202A-202E comprise five blocks, where each block (e.g. blocks 210A, 211A, 212A, 213A and 214A for the down-sampling blocks in encoder 206A) learns shape features at a different spatial resolution, enabling the encoder-decoder networks to learn shape features with both global and local context, across multiple mesh resolutions. This is enabled by including mesh down-sampling (and up-sampling for the decoder 216 networks) operations in each of the five blocks of the encoder 206 and decoder 216 networks respectively. The tables (1-5) below summarise the architectural details of each encoder-decoder network pair in each of the five gcVAEs that may be used to model the LV, RV, LA, RA AND AR. The architecture of residual graph convolution down-sampling blocks and the residual graph convolution up-sampling blocks used in the encoder and decoder networks, respectively, in each of the five gcVAEs, may be structurally similar, for example with only the down- and up- sampling factors used varying across the different gcVAEs and are depicted in Figures 4 and 5, respectively. According to an example, the number of feature channels (i.e. the number of different learnable Chebyshev convolution filters/operations) used within each graph convolution layer in the residual graph convolution down-sampling blocks, from blocks 1 to 5 are: 16, 32, 32, 64 and 64, and the number of feature channels used within each graph convolution layer in the residual graph convolution up-sampling blocks, from blocks 1 to 5 are: 64, 64, 32, 32, and 16. Other numbers of feature channels may also be used. The mesh down-sampling factors used across down-sampling blocks 1 to 5 were different for encoder networks in each part-specific gcVAE and are detailed as follows - left ventricle: [4,4,4,6,6]; right ventricle: [4,4,4,6,6]; left atrium: [4,4,4,5,5]; right atrium: [4,4,4,5,5]; and aorta: [4,4,4,4,4], where, each number within brackets corresponds to the down-sampling factor used in residual graph convolution down-sampling blocks from block 1 to 5, for each part-specific gcVAE. Similarly, the up-sampling factors used across up-sampling bocks 1 to 5 were different for decoder networks in each part-specific gcVAE and are detailed as follows - left ventricle: [6,6,4,4,4]; right ventricle: [6,6,4,4,4]; left atrium: [5,5,4,4,4]; right atrium: [5,5,4,4,4]; and aorta: [4,4,4,4,4], where, each number within brackets corresponds to the up-sampling factor used in residual graph convolution up-sampling blocks from block 1 to 5, for each part-specific gcVAE. Note, that at each corresponding resolution level between the encoder and decoder the resampling factor is the same, i.e. for the decoder the order is reversed as it goes from a low resolution to a high resolution (while the encoder goes from a high resolution to a low resolution - i.e. downsamples). The number of feature channels and mesh resampling factors used in the disclosed part-specific gcVAEs were determined empirically to be the best for the example application (i.e. cardiac modelling) and training data used. Their values may be changed and varied given different training data for the same or different applications in generative modelling of multi-part or multi-organ shape assemblies. All residual graph convolution down-sampling and up-sampling blocks used throughout all networks in the disclosed system also contain instance normalisation layers, a residual connection and an exponential linear activation unit organised as shown in Figures 4 and 5.

Table 1: An example network architecture for the gcVAE 202A applied to left ventricular (LV) shapes. $V_{LV}$ represents the number of nodes/vertices present in each LV mesh/unstructured graph, for all samples in the training, validation and test sets used to train and evaluate the disclosed system.

| Encoder | Decoder |
|---|---|
| Input: LV mesh/unstructured graph ($x_i$), size: ($V_{LV} \times 3$) | Input: Latent variables vector ($z_i$), size: (16x1) vector |
| Residual Graph Convolution Downs-ampling Blocks 1 - 5 | Residual Graph Convolution Up-sampling Blocks 1-5 |
| Fully connected output layer | Chebyshev convolution layer, Size: size: ($V_{LV} \times 3$) |
| Output: Latent variables vector ($z_i$), size: (16x1) vector | Output: Reconstructed LV mesh/unstructured graph (r) |

Table 2: An example network architecture for the gcVAE 202B applied to right ventricular (RV) shapes. $V_{RV}$ represents the number of nodes/vertices present in each RV mesh/unstructured graph, for all samples in the training, validation and test sets used to train and evaluate the disclosed system.

| Encoder | Decoder |
|---|---|
| Input: RV mesh/unstructured graph ($x_i$), size: (VRVX 3) | Input: Latent variables vector ($z_i$), size: (12x1) vector |
| Residual Graph Convolution Down sampling Blocks 1 - 5 | Residual Graph Convolution Up sampling Blocks 1 - 5 |
| Fully connected output layer | Chebyshev convolution layer, Size: size: ($V_{RV} \times 3$) |
| Output: Latent variables vector ($z_i$), size: (12x1) vector | Output: Reconstructed RV mesh/unstructured graph (r) |

Table 3: An example network architecture for the gcVAE 202C applied to left atrial (LA) shapes. $V_{LA}$ represents the number of nodes/vertices present in each LA mesh/unstructured graph, for all samples in the training, validation and test sets used to train and evaluate the disclosed system.

| Encoder | Decoder |
|---|---|
| Input: LA mesh/unstructured graph ($x_i$), size: (VLAX 3) | Input: Latent variables vector ($z_i$), size: (16x1) vector |
| Residual Graph Convolution Down-sampling Blocks 1-5 | Residual Graph Convolution Up-sampling Blocks 1-5 |
| Fully connected output layer | Chebyshev convolution layer, Size: size: ($V_{LA} \times 3$) |
| Output: Latent variables vector ($z_i$), size: (16x1) vector | Output: Reconstructed LA mesh/unstructured graph (r) |

Table 4: An example network architecture for the gcVAE 202D applied to right atrial (RA) shapes. $V_{RA}$ represents the number of nodes/vertices present in each RA mesh/unstructured graph, for all samples in the training, validation and test sets used to train and evaluate the disclosed system.

| Encoder | Decoder |
| --- | --- |
| Input: RA mesh/unstructured graph ($x_i$), size: ($V_{RA} \times 3$) | Input: Latent variables vector ($z_i$), size: (12x1) vector |
| Residual Graph Convolution Down sampling Blocks 1-5 | Residual Graph Convolution Up sampling Blocks 1-5 |
| Fully connected output layer | Chebyshev convolution layer, Size: size: ($V_{RA} \times 3$) |
| Output: Latent variables vector ($z_i$), size: (12x1) vector | Output: Reconstructed RA mesh/unstructured graph (r) |

Table 5: An example network architecture for the gcVAE 202E applied to aortic root (AR) shapes. $V_{AR}$ represents the number of nodes/vertices present in each AR mesh/unstructured graph, for all samples in the training, validation and test sets used to train and evaluate the disclosed system.

| Encoder | Decoder |
| --- | --- |
| Input: AR mesh/unstructured graph ($x_i$), size: ($V_{AR} \times 3$) | Input: Latent variables vector ($z_i$), size: (8x1) vector |
| Residual Graph Convolution Down sampling Blocks 1-5 | Residual Graph Convolution Up sampling Blocks 1-5 |
| Fully connected output layer | Chebyshev convolution layer, Size: size: ($V_{AR} \times 3$) |
| Output: Latent variables vector ($z_i$), size: (8x1) vector | Output: Reconstructed AR mesh/unstructured graph (r) |

As can be seen clearly in Figure 2, the independent generator 200 is called independent, since it maintains separation between the processing of each gcVAE (202A-E), where each gcVAE is trained independently with their own training loop.

Figure 3 shows an example dependent generator 300 for use in the system of Figure 1, according to an example of the disclosure. The example dependent generator 300 is a single generative model formulated as a graph-convolutional multi-channel variational autoencoder (gcmcVAE), comprising multiple encoder-decoder pairs, one per part being modelled (i.e. it may be thought of as one gcmcVAE with multiple branches, as opposed to the multiple independent gcVAEs in the independent generator of Figure 2), and so comprises N=5 pairs of encoder-decoder networks that correspond to the five cardiac structures of interest (e.g. LA, RA, LV, RV, AR). The encoder 306 comprises a plurality of residual graph convolution down-sampling (RGCDS) blocks (e.g. 310A-314A), and the decoder comprises a plurality of residual graph convolution up-sampling (RGCUS) blocks (e.g. 318A-322A). The RGCDS and RGCUS blocks are again the same as those discussed in more detail with respect to Figures 4 and 5 below. As shown in Figure 3, each of the 5 encoder-decoder pairs in the gcmcVAE (denoted by the letters A-E) takes as input a mesh for the respective part, e.g. LV mesh 301A, which is down-sampled in the encoder 306, to form a fully connected layer Latent vector 315A. In the example of Figure 3, each of the five different sub-models have the same vector size applied = 12x1, but other sizes may be used instead. Unlike the independent generator 200 however, each latent vector 315A-E corresponding to each of the five different sub-models in the dependent generator 300 is implemented using the same fixed size (e.g. = 12x1). This is because, in the dependent generator 300, each latent vector is no longer independent as in the gcVAEs (202A-E) of the independent generator 200, but are shared or 'dependent' on each other. In other words, each encoder (306A-E) in the gcmcVAE of the dependent generator 300 maps the inputted mesh for the respective part to a shared latent vector (315A-E), where the latent vectors (315A-E) are considered to be shared as by design the outputs of each encoder (306A-E) are formulated as different approximations of the same shared latent vector (or as approximations of the same shared hidden representation across all input channels of information). This formulation of the outputs of each encoder (306A-E) being approximations of the same shared latent vector is enforced by imposing a constraint on the loss function (described in subsequent paragraphs) used to train the gcmcVAE, wherein, each encoder's (306A-E) approximation of the shared latent vector is passed as input to all decoders (316A-E) (e.g. via multiplexing layer 317) and each decoder (316A-E) is used to reconstruct all channels of information or meshes of all parts inputted to the encoders (306A-E). In other words, the encoders (306A-E) in the gcmcVAE are channel or part-specific, which output different approximations of the shared latent vector (315A-E), while, the decoders (316A-E) operate across channels or parts and simultaneously output/reconstruct all channels of information or meshes of all parts. As the outputs of all encoders (306A-E) are used as inputs to all decoders (316A-E), the input size for each decoder (316A-E) must be the same. Hence, for the dependent generator 300 the latent vectors (315A-E) output by each encoder (306A-E), have the same size.

In more detail - the input to each encoder-decoder network pair in the gcmcVAE is a mesh/unstructured graph 301A-E representing the relevant anatomical part or organ's shape, which together constitute the multi-part/multi-organ shape assembly to be synthesised by the disclosed generative shape modelling system.

All encoder-decoder network pairs 302A-E in the gcmcVAE are trained jointly to learn a shared/common latent representation across all anatomical parts and/or organs of interest in the input data. A mcVAE is an extension to the VAE which jointly analyses heterogeneous data by projecting observations from different input sources/channels to a shared/common latent representation. The training procedure for the mcVAE to learn a shared latent representation across multiple input sources/channels of data is similar to the VAE in that, it is achieved by approximating the posterior distribution of the shared latent variables given the observed heterogeneous input data. This is achieved by jointly optimising all N constituent encoder-decoder network pairs 302A-E (which represent the N input sources/channels of data, or the N anatomical parts' and/or organs' shapes in the case of the gcmcVAE in the present invention) to maximise the associated evidence lower bound (ELBO) of the observed heterogeneous input data.

To train the gcmcVAE consider the data sets denoted $X^{n=1...N} = \{x^n_i\}_{i=1...D}$ where, each $x^n_i$ denotes the shape of an anatomical part (e.g. left ventricle) of an individual represented as a computational mesh. Each mesh/unstructured graph is represented by a list of 3D points/spatial coordinates (referred to as vertices) defining the boundary of the anatomical part/shape and an adjacency matrix defining vertex connectivity. Each individual patient's data in $X^n$ is denoted by subscript $i$, where, $\{i=1...D\}$ and D is the number of individuals' data is included in $X^n$. Each $X^n$ is the data input to each of the $\{n=1...N\}$ pairs of encoder-decoder networks in the gcmcVAE, which in this example N=5. The D individuals whose data constitute each $X^n$ do not have to be the same, i.e. training data samples $\{x^n_i\}_{i=1...D}$ in each $X^n$ may come from the same or different individuals. In other words, the data sets $X^n$ used to train each gcmcVAE can comprise partially-overlapping or completely overlapping data, where - partially-overlapping data means that some proportion of samples in each $X^n$ are obtained from the same individuals; and completely overlapping data means that all samples in each $X^n$ are obtained from the same individuals. The dependent generator 300 cannot be trained with non-overlapping data, unlike the independent generator 200. The gcmcVAE by design learns a shared/common latent representation across all input channels of information/anatomical parts and does so by exploiting the correlations that exist within the provided inputs. Hence, the dependent generator uses the inputs obtained from different patients/patient populations to have some overlap in information (i.e. to be at least partially overlapping). Each encoder network in the gcmcVAE takes a mesh/unstructured graph-based representation of shape for a given anatomical part or organ as input i.e. $x^n_i$ (refer to Figure 3) and all encoder networks in the gcmcVAE jointly map these high-dimensional representations of shapes to a shared/common low-dimensional vector or latent representation (denoted zi) or in other words, each encoder network outputs a different approximation to the shared latent vector zi, given its corresponding anatomical part or organ $x^n_i$ as input. The approximations to the shared/common latent representation resulting from each anatomical part or organ shape being passed through its corresponding encoder network are subsequently provided as inputs to all decoder networks in the gcmcVAE. Each decoder network in turn maps back or reconstructs the original mesh for the anatomical part or organ inputted to its corresponding encoder, using as input each of the latent vector approximations output by each of the encoder networks. In other words, given that there are N parts in the shape assembly for each individual patient's data used to train the gcmcVAE, each of the N encoders in the gcmcVAE output N approximations to the latent representation shared across all N parts. And, each of the N decoders in the gcmcVAE take as inputs all of the N approximations of the shared latent representation and output/reconstruct N versions/approximations of the shape for their corresponding part (i.e. for the part inputted to the encoder that corresponds to each of the N decoders). During training, the N different reconstructions outputted for each part by its corresponding decoder result in N different losses that are aggregated/summed together and used to guide the training of the gcmcVAE. By training the gcmcVAE on the data sets $X^{n=1...N}$ a shared/common latent representation for the data sets $X^{n=1...N}$ is discovered.

Each of the N=5 encoder-decoder network pairs in the gcmcVAE are trained jointly using their corresponding input training data sets $X^{n=1...N}$, to learn a shared/common latent representation from which the observed data sets $X^{n=1...N}$ are assumed to be generated, by approximating the true but intractable posterior distribution of the latent variables using variational inference. This is achieved by optimising the evidence lower bound (ELBO) of the observed data sets $X^{n=1...N}$ with respect to the parameters of all encoder-decoder network pairs in the gcmcVAE. In this formulation, each encoder network in the gcmcVAE approximates the posterior distribution (denoted $q_n(z \mid X^n)$), or in other words outputs an approximation to the shared latent representation, given its input data set $X^n$ and each corresponding decoder network approximates the data likelihood (denoted $p_n(X^n \mid z)$) given the shared latent vector z , using each of the N approximations to z output by each of the N encoder networks in the gcmcVAE. As each of the N=5 input channels (i.e. anatomical part/organ shape) in the gcmcVAE is assumed to be conditionally independent from all the others given the shared/common latent representation z (i.e. the input channels are considered to be independent from each other, but dependent on the shared latent variables z), the joint data likelihood across all input channels is formulated as $p(X \mid z)$ given by:

$$p(\mathbf{X} \mid \mathbf{z}) = \prod_{n=1}^{N} p_n(\mathbf{X}^n \mid \mathbf{z}) \quad (6)$$

To train the gcmcVAE, given an input sample $\mathbf{s}_i$ where, $\mathbf{s}_i = \{\mathbf{x}_i\}^{n=1...N}$ represents a set of shapes (i.e. here the shapes belong to one of the five cardiac structures of interest), each individual shape $\mathbf{x}^n_i$ in $\mathbf{s}_i$ is passed as input to its corresponding encoder network. All $\mathbf{x}^n_i$ in each $\mathbf{s}_i$ come from the same individual represented by subscript 'i'. Not all five cardiac structures need to be present in each sample $\mathbf{s}_i$ used to train the gcmcVAE, i.e. the gcmcVAE can be trained with partially-overlapping data. All five encoder networks in the gcmcVAE map their corresponding inputs $\mathbf{x}^n_i$ (if $\mathbf{x}^n_i$ is present in $\mathbf{s}_i$) to approximations of a unique latent representation $\mathbf{z}_i$ which in turn is used as input by all decoder networks to reconstruct the shapes of their corresponding anatomical parts or organs (denoted $\mathbf{r}^n_i$). Thus, the gcmcVAE comprises shape-specific encoder networks that map each shape to a shared/common latent representation, and cross-shape decoder networks that can simultaneously reconstruct all shapes, given the shared/common latent representation as input. This enables the dependent generator 300, once trained, to 'complete/impute' missing parts/channels of information conditioned on the provided inputs. Specifically, this imputation or conditional generation of missing parts/channels of information, given some incomplete input parts/channels of information, is enabled by training each decoder in the gcmcVAE to reconstruct its corresponding part using approximations to the shared latent space outputted by all encoders in the gcmcVAE (i.e. by training the decoders for cross-shape/cross-channel synthesis). Additionally, the design constraints in the gcmcVAE that facilitate this cross-shape training and subsequent cross-shape synthesis/imputation of missing parts/channels of information are the fixed size latent vectors outputted by all encoder networks and correspondingly the fixed size input vectors expected by all decoder networks; and the formulation of the loss function used to train the gcmcVAE (described in the next paragraph). As the latent representation learned across all input parts/channels is shared, following training of the gcmcVAE, multiple parts/channels can be reconstructed using the latent representation resulting from a single input part/channel of information. Additionally, the shared/common latent representation can be sampled from the assumed prior distribution over the latent variables (i.e. $p(\mathbf{z}) = \mathcal{N}(\mathbf{0}, \mathbf{I})$) to synthesise new/virtual shapes of parts (not observed in the training data), by passing the sampled latent representation through all decoder networks simultaneously.

The gcmcVAE is trained by jointly optimising all its constituent pairs of encoder-decoder networks to maximise the evidence lower bound (ELBO) of the observed input data sets $\{\mathbf{X}^n\}_{n=1...5}$. The ELBO is formulated as a loss function comprising a summation of the expected joint negative log-likelihood of the data (denoted $L_{recon}$), the Kullback-Leibler divergence of the approximate posterior distribution of the shared latent variables $q(\mathbf{z} \mid \mathbf{X}^n)$ from the assumed prior distribution $p(\mathbf{z})$ over the shared latent variables (denoted $L_{KL}$), and an additional regularisation loss (denoted $L_{regular}$). The key difference between the gcVAEs (202A-E) in the independent generator 200 and the gcmcVAE (302A-E) in the dependent generator 300 is in the formulation of the ELBO used to train their constituent encoder and decoder networks. The gcVAEs (202A-E) in the independent generator 200 consider each anatomical part or organ shape of interest to be completely independent and hence each gcVAE learns an independent/unique latent representation for its corresponding part. The gcmcVAE (302A-E) however, assumes all parts or organ shapes in the shape assembly are conditionally independent, or in other words each part is dependent on a shared latent representation, and thus learns a shared latent representation across all parts. The formulation of the ELBO loss function used to train the gcmcVAE (302A-E) in the dependent generator 300 is thus derived by imposing a constraint to minimise the Kullback-Leibler divergence between each approximation of the posterior distribution over the shared latent variables $q_n(\mathbf{z} \mid \mathbf{X}^n)$ outputted by each encoder (306A-E) network and the unknown target posterior distribution $p(\mathbf{z} \mid \mathbf{X}^1, \mathbf{X}^2, ..., \mathbf{X}^n)$. Conversely, each gcVAE (202A-E) in the independent generator 200 is trained by maximising a separate ELBO loss function, independently from the other gcVAEs. Here, each ELBO loss function is formulated such that the Kullback-Leibler divergence between the approximate posterior distribution over the latent variables $q(\mathbf{z}^n \mid \mathbf{X}^n)$ outputted by each encoder (206A-E) network specific to each gcVAE, and the unknown target posterior distribution $p(\mathbf{z}^n \mid \mathbf{X}^n)$, is minimised independently of the other approximate posterior distributions and latent variables outputted by the encoders in other gcVAEs. As in the case of the gcVAEs in the independent generator 200, directly minimising the Kullback-Leibler divergence between each approximated posterior distribution over the shared latent variables $q_n(\mathbf{z} \mid \mathbf{X}^n)$ and the true unknown (or target) posterior distribution over the shared latent variables $p(\mathbf{z} \mid \mathbf{X}^1, \mathbf{X}^2, ..., \mathbf{X}^n)$, to train the gcmcVAE in the dependent generator 300, is intractable (cannot be computed/estimated). This is because the target posterior distribution $p(\mathbf{z} \mid \mathbf{X}^1, \mathbf{X}^2, ..., \mathbf{X}^n)$ is unknown and itself intractable in the present setting. Instead, the ELBO loss function used to train the gcmcVAE (302A-E) is formulated by factorising (expressing) the target posterior distribution as a product of the joint data likelihood $p(\mathbf{X} \mid \mathbf{z})$ across all parts (refer to equation 6) and the assumed prior distribution over the shared latent variables $p(\mathbf{z})$, using Bayes' theorem. As a result, the Kullback-Leibler divergence loss term $L_{KL}$ reduces to minimising the divergence between each approximation of the posterior distribution over the shared latent variables $q_n(\mathbf{z} \mid \mathbf{X}^n)$ outputted by each encoder (306A-E) network in the gcmcVAE and the assumed prior distribution over the shared latent variables p(z). Or, in other words, to learn a shared latent representation across all input parts, the gcmcVAE is trained by minimising the approximation of the posterior distribution over the shared latent variables outputted by each encoder (306A-E) network in the gcmcVAE from the target posterior distribution, with the underlying hypothesis that each input part carries with it useful information regarding the shared latent representation for itself and all other parts in the shape assembly. The overall ELBO loss function ($L_{total}$) used to train the gcmcVAE in the dependent generator 300 is formulated the same way as $L_{total}$ used to train each gcVAE in the independent generator 200, however, the individual loss terms in $L_{total}$ are formulated differently for the dependent generator 300. The reconstruction loss $L_{recon}$ (representing the joint negative log-likelihood of the data) is computed as the sum of the vertex-wise $L_1$- norms evaluated between each reconstructed ($r_i^{n|n'}$) shape/mesh output by each decoder network and the original shape/mesh ($x^n_i$) input to the corresponding encoder network. Here, as each of the N encoders (306A-E) output an approximation to the shared latent vector (z), each decoder (316A-E) receives as input N approximations to $z$, and in turn each decoder is trained to reconstruct its corresponding part/mesh (i.e. the part/mesh inputted to its corresponding encoder) using each of the N approximations to z outputted by each of the N encoder networks (306A-E). Hence, $r_i^{n|n'}$ denotes the part/mesh 'n' reconstructed by one of the n={1...N} decoders (316A-E), for sample 'i', given an approximation to the shared latent vector n' outputted by each of the n = {1...N} encoders (306A-E) in the gcmcVAE. As there are N encoders, n' = {1...N} different approximations to the shared latent space z are passed as inputs to each decoder (316A-E), resulting in N different reconstructions of the $n^{th}$ part for the $i^{th}$ sample. Consequently, N different reconstruction loss terms are evaluated for each part in the shape assembly and are aggregated/summed together to formulate $L_{recon}$ as:

$$L_{recon} = \sum_{n=1}^{N} \sum_{n'=1}^{N} \left\| \mathbf{x}_i^n - \mathbf{r}_i^{n\,|\,n'} \right\|_1 \quad (7)$$

The Kullback-Leibler divergence loss term $L_{KL}$ is computed as the sum of the divergence between the posterior distribution over the shared/common latent variables approximated by each encoder network, i.e. $q_n(z \mid X^n)$ and the assumed prior distribution over the shared latent variables. The prior distribution over the latent variables is assumed to be a centered isotropic multivariate Gaussian distribution, i.e. $p(\mathbf{z}) = \mathcal{N}(\mathbf{0}, \mathbf{I})$. An isotropic multi-variate Gaussian prior is assumed for computational simplicity, and other types of prior distributions may be used within the disclosed system, for example, a Dirichlet distribution, a Gaussian Mixture Model, a Gaussian process, a Dirichlet process, among others. As stated previously, the gcmcVAE is trained to learn a shared latent representation across all parts/input channels of information by formulating the reconstruction loss term $L_{recon}$ as shown in equation 7, and by minimising the divergence of each approximated posterior distribution over the shared latent variables $q_n(z \mid X^n)$, corresponding to each input part/channel of information and its specific encoder (306A-E), from the assumed prior distribution p(z). Additionally, the training gcmcVAE using the formulation for $L_{recon}$ shown in equation 7, enables cross-shape/cross-channel imputation of missing parts/channels of information (i.e. given partially-overlapping data as inputs, the gcmcVAE once trained can be used to impute/conditionally synthesise the missing parts/channels of information). Put another way, training the gcmcVAE using this formulation of $L_{recon}$ allows reconstruction of multi-channel/multi-part data given single channel/part inputs.

The regularisation loss term $L_{regular}$ in $L_{total}$ for the dependent generator is formulated as:

$$L_{regular} = \sum_{n=1}^{N} w_1 L_{laplacian}^n + w_2 L_{edge}^n \quad (8)$$

where, $L_{laplacian}^n$ and $L_{edge}^n$ are the Laplacian smoothness loss and the edge length loss estimated for each shape/mesh output by each decoder, i.e. $r^n_i$. Each of the {n=1...N} Laplacian smoothness and edge length loss terms (in this example N=5) are formulated in the same way as used for each gcVAE in the independent generator 200. As with each gcVAE in the independent generator 200, here, $w_1$ and $w_2$ denote the weights that are multiplied with each $L_{laplacian}^n$ and $L_{edge}^n$ term, respectively, in order to balance their influence on the regularisation loss term and the overall training process, relative to the other terms in the overall loss ($L_{total}$).

The gcmcVAE is trained by minimising the overall loss function $L_{total}$ with respect to the parameters of all its constituent encoder-decoder network pairs. The parameters of all encoder-decoder network pairs in gcmcVAE are learned iteratively via the error backpropagation algorithm.

There now follows an overview of an example network architecture that may be used for the gcmcVAE 306A-E in the dependent generator 300 as depicted in Figure 3. Five pairs of encoder-decoder networks are used in the current implementation of the gcmcVAE, corresponding to the five cardiac structures of interest, however, the number of pairs of encoder-decoder networks can be varied to suit different applications (i.e. where the number of structures that constitute the multi-part shape assemblies of interest are less/greater than five). The encoder 306 and decoder 316 networks in all five pairs (A-E) comprise five blocks each, where each block (e.g. blocks 310A, 311A, 312A, 313A and 314A for the down-sampling blocks in encoder 306A) learns shape features at a different spatial resolution, enabling the encoder-decoder networks to learn shape features with both global and local context, across multiple mesh resolutions. This is enabled by including mesh down-sampling and up-sampling operations in each of the five blocks of the encoder 306 and decoder 316 networks respectively. Table 6 below summarises the architectural details of each encoder-decoder network pair 302A-E in the gcmcVAE used to model the five cardiac structures of interest, namely, the LV, RV, LA, RA and AR. The architecture of residual graph convolution down-sampling blocks (310-314) and the residual graph convolution up-sampling blocks (318-322) used in the encoder and decoder networks, respectively, in all five pairs, were similar (i.e. only the down- and up-sampling factors used varied across different pairs of encoder-decoder networks) and are depicted in Figure 4. The number of feature channels used within each graph convolution layer in the residual graph convolution down-sampling blocks, from blocks 1 to 5 are: 16, 32, 32, 64 and 64, respectively. Similarly, the number of feature channels used within each graph convolution layer in the residual graph convolution up-sampling blocks, from blocks 1 to 5 are: 64, 64, 32, 32, and 16, respectively. The mesh down-sampling factors used across down-sampling blocks 1 to 5 were different for encoder networks in each encoder-decoder network pair included in the gcmcVAE. These are described according to the cardiac structure each encoder network accepts as input, as follows - left ventricle: [4,4,4,6,6]; right ventricle: [4,4,4,6,6]; left atrium: [4,4,4,5,5]; right atrium: [4,4,4,5,5]; and aorta: [4,4,4,4,4], where, each number within brackets corresponds to the down-sampling factor used in residual graph convolution down-sampling blocks from block 1 to 5, in each encoder-decoder network pair. Similarly, the up-sampling factors used across up-sampling bocks 1 to 5 were different for decoder networks in each encoder-decoder network pair included in the gcmcVAE. These are described according to the cardiac structure reconstructed/output by each decoder network, as follows - left ventricle: [6,6,4,4,4]; right ventricle: [6,6,4,4,4]; left atrium: [5,5,4,4,4]; right atrium: [5,5,4,4,4]; and aorta: [4,4,4,4,4], where, each number within brackets corresponds to the up-sampling factor used in residual graph convolution up-sampling blocks from block 1 to 5, in each encoder-decoder network pair.

Table 6: An example network architecture for the gcmcVAE 302A-302E that may be used in the dependent generator 300 to learn variability in shapes of all five cardiac structures of interest.

| Encoder-1 | Encoder-2 | Encoder-3 | Encoder-4 | Encoder-5 |
|---|---|---|---|---|
| Input: LV mesh/ unstructured graph ($x_i$), size: (VLVX 3) | Input: RV mesh/ unstructured graph ($x_i$), size: (VRVX 3) | Input: LA mesh/ unstructured graph ($x_i$), size: (VLAX 3) | Input: RA mesh/ unstructured graph ($x_i$), size: ($V_{RA} \times 3$) | Input: AR mesh/ unstructured graph ($x_i$), size: ($V_{AR} \times 3$) |
| Residual Graph Convolution Down-sampling Blocks 1-5 | Residual Graph Convolution Down-sampling Blocks 1-5 | Residual Graph Convolution Down-sampling Blocks 1-5 | Residual Graph Convolution Down-sampling Blocks 1-5 | Residual Graph Convolution Down-sampling Blocks 1-5 |
| Fully connected output layer | Fully connected output layer | Fully connected output layer | Fully connected output layer | Fully connected output layer |
| Output: Latent variables vector ($z_i$), size: (12x1) vector | Output: Latent variables vector ($z_i$), size: (12x1) vector | Output: Latent variables vector ($z_i$), size: (12x1) vector | Output: Latent variables vector ($z_i$), size: (12x1) vector | Output: Latent variables vector ($z_i$), size: (12x1) vector |
| Multiplexing layer allowing output of Encoders 1 to 5 to input into each of Decoders 1 to 5 | | | | |
| Decoder-1 | Decoder-2 | Decoder-3 | Decoder-4 | Decoder-5 |
| Input: Latent variables vector ($z_i$), size: (12x1) vector | Input: Latent variables vector ($z_i$), size: (12x1) vector | Input: Latent variables vector ($z_i$), size: (12x1) vector | Input: Latent variables vector ($z_i$), size: (12x1) vector | Input: Latent variables vector ($z_i$), size: (12x1) vector |
| Residual Graph Convolution Up-sampling Block s1-5 | Residual Graph Convolution Up-sampling Blocks 1-5 | Residual Graph Convolution Up-sampling Blocks 1-5 | Residual Graph Convolution Up-sampling Blocks 1-5 | Residual Graph Convolution Up-sampling Blocks 1-5 |
| Chebyshev convolution layer, Size: ($V_{LV} \times 3$) | Chebyshev convolution layer, Size: ($V_{RV} \times 3$) | Chebyshev convolution layer, Size: ($V_{LA} \times 3$) | Chebyshev convolution layer, Size: ($V_{RA} \times 3$) | Chebyshev convolution layer, Size: ($V_{AR} \times 3$) |

(continued)

| Multiplexing layer allowing output of Encoders 1 to 5 to input into each of Decoders 1 to 5 | | | | |
|---|---|---|---|---|
| Decoder-1 | Decoder-2 | Decoder-3 | Decoder-4 | Decoder-5 |
| Output: Reconstructed LV mesh/unstructured graph ($r_i$) | Output: Reconstructed RV mesh/unstructured graph ($r_i$) | Output: Reconstructed LA mesh/unstructured graph ($r_i$) | Output: Reconstructed RA mesh/unstructured graph ($r_i$) | Output: Reconstructed AR mesh/unstructured graph ($r_i$) |

The dependent generator 300 is called dependent because a shared latent representation is assumed across all parts/input channels of information, such that the output of each decoder (316A-E) in the gcmcVAE is dependent on the shared latent representation across all parts. As such, the dependent generator 300 is trained in a singular training loop by simultaneously optimising all constituent encoder (306A-E) and decoder (316A-E) networks using a single loss function. This is in contrast with the independent generator 200 wherein, each gcVAE (202A-E) is trained independently for each part, and the latent representation learned for each part is independent of those learned for the other parts. Additionally, the independent generator 200 is trained with separate/independent training loops for each constituent gcVAE (202A-E), using separate/independent loss functions.

The foregoing description has referred to residual graph convolutional down-sampling (RGCDS) blocks and residual graph convolutional up-sampling (RGCUS) blocks, which will now be described with reference to Figures 4 and 5. Examples use these residual graph convolutional down-sampling/up-sampling blocks to strengthen the feature exchange efficiency between vertices (i.e. nodes in the mesh) and mitigate the issue of vanishing gradients when training the networks.

Figure 4 shows an example architecture of a residual graph convolutional down-sampling block for use in all graph neural networks in the system of Figure 1, according to an example of the disclosure. The RGCDS block takes as input a set of meshes, e.g. the outputs from the preceding RGCDS in the encoder networks used within the disclosed independent generator and dependent generator) 401, and applies a Chebyshev graph convolution 410, and then applies an Instance Normalisation process 420, before applying an Exponential Linear Unit function 430. Note, in some examples, the input 401 may be a single mesh. Next, another Chebyshev graph convolution 440 and Instance Normalisation process 450 may be applied before the result is added together with a separate path 415 (i.e. residual branch) from the original Chebyshev graph convolution 410. The summed result then may have another an Exponential Linear Unit function 470 applied, before a final Mesh Pooling Layer function is applied, and the result outputted 491. As such, each residual block comprises at least one graph convolution layer that extract features using the provided inputs to the block, and a residual connection that combines the output of the first and second graph convolution layers through summation. Chebyshev graph convolution operations are used throughout this example due to their strictly localised filters that enables learning of multi-scale hierarchical patterns when combined with mesh pooling operations, and low computational complexity. Each graph convolution layer is followed with an instance normalization layer and an exponential linear unit (ELU) for activation. Additionally, the instance normalisation layer is used in the residual branch to ensure similarity in feature statistics relative to the output of the second graph convolution layer in the block.

Figure 5 shows an example architecture of a residual graph convolutional up-sampling block for use in all graph neural networks in the system of Figure 1, according to an example of the disclosure;

The RGCUS block is similar to the RGCDS block of Figure 4, but the inverse. The RGCUS block is used in the decoder networks, within the disclosed part-aware generative model 102. As shown, the RGCUS takes as input 501 either the latent vector output from an encoder network or the output from the preceding RGCUS block in the same decoder network, and applies a Mesh Up-sampling function 510, followed by a Chebyshev graph convolution 520, and a first Instance Normalisation process 530, before applying an Exponential Linear Unit function 540, which may be followed by another Chebyshev graph convolution 550 and Instance Normalisation process 560. The result is then added together with a separate path 515 (i.e. residual branch) from the original Chebyshev graph convolution 520. Finally, the summed result then may have another an Exponential Linear Unit function 570 applied, before the result outputted 591. As such, each residual block first processes the provided inputs by up-sampling them using a mesh up-sampling layer, and comprises two graph convolution layers that extract features outputs of the mesh up-sampling layer, and a residual connection that combines the output of the first and second graph convolution layers through summation. Chebyshev graph convolution operations are used throughout this example due to their strictly localised filters that enables learning of multi-scale hierarchical patterns when combined with mesh pooling operations, and low computational complexity. Each graph convolution layer is followed with an instance normalization layer and an exponential linear unit (ELU) for activation. Additionally, the instance normalisation layer is used in the residual branch to ensure similarity in feature statistics relative to the output of the second graph convolution layer in the block.

Once the part-aware generative module 110 is trained, using either the independent 200 or dependent 300 generator, all anatomical parts and/or organs in the shape assembly can be synthesised. Note, there are two ways to train the part-aware generative model 102. Either using an independent generator 200 (e.g. of Figure 2) or a dependent generator 300 (e.g. of Figure 3). For the independent generator 200, any of non-overlapping, partially-overlapping and completely overlapping data may be used. For the dependent generator 300, only partially-overlapping or completely-overlapping data may be used. This synthesis can be done by sampling new latent vectors from the learned latent distributions, i.e. in the case of the independent generator 200, new latent vectors may be sampled from each gcVAE to synthesise each corresponding anatomical part/organ. While, for the dependent generator 300, a new latent vector may be sampled form the shared/common latent distribution learned by the gcmcVAE across all anatomical parts/organs of interest. The sampled latent vectors may be passed as inputs to their corresponding decoder networks to reconstruct/synthesise new anatomical parts'/organs' shapes, i.e. latent vectors sampled from each gcVAE in the independent generator 200 may be passed to their corresponding decoder networks, independent of each other. Meanwhile, the latent vectors sampled from the gcmcVAE may be all passed simultaneously to all constituent decoder networks (i.e. in the dependent generator all decoder networks may receive the same sampled latent vectors as inputs). Using either the independent 200 or dependent 300 generator, anatomical parts/organs may be synthesised through their corresponding decoder networks separately. Hence, synthesised parts initially may not be spatially organised/aligned into shape assemblies representative of realistic native anatomy (e.g. synthesised parts may have significant intersections/overlaps between each other, which does not respect naturally occurring variations in anatomy). Therefore, a spatial composition network may then be applied by the spatial composition module 120 of Figure 1 to learn the spatial relationships between parts in the shape assembly and estimate the spatial transformations necessary to compose (compose may also be referred to as organise or align) the synthesised individual parts into anatomically meaningful (i.e. realistic) shape assemblies (such as the whole heart shape assembly of interest in the present example). An example of the spatial composition network of the present disclosure takes individual anatomical parts'/organs' meshes synthesised using either the independent or dependent generator as input, and estimates both affine and non-rigid (also referred to as deformable) transformations to spatially organise the inputs parts into multi-part/multi-organ shape assemblies (i.e. virtual chimeras) that are representative of native anatomy. According to the example, the inputs to the spatial composition network include mesh/graph-based representations of shapes for the left ventricle, right ventricle, left atrium, right atrium and aortic root, and the spatially composed outputs are cardiac virtual chimeras (also referred to as whole heart shape assemblies).

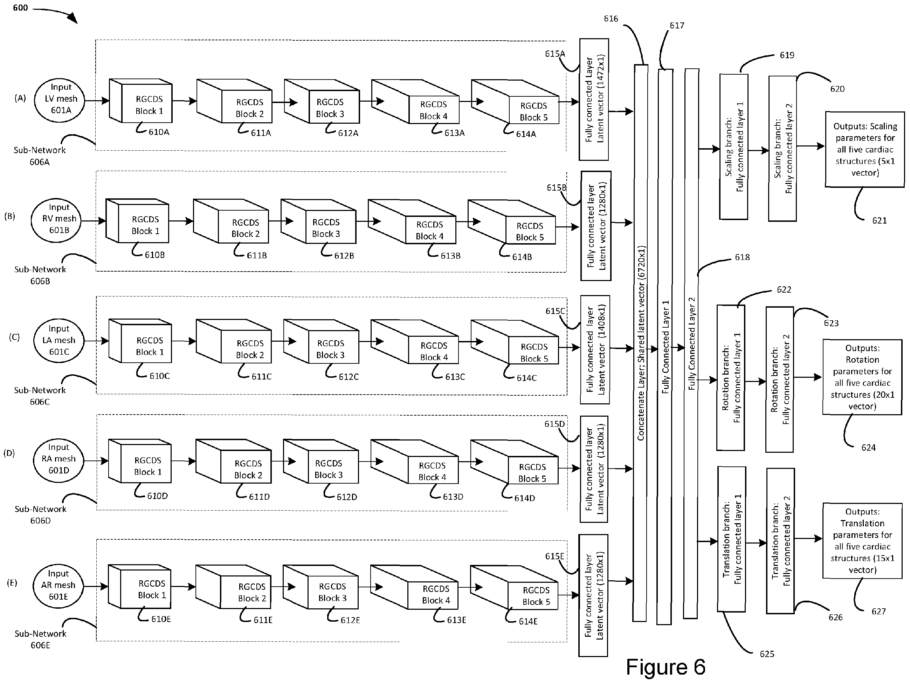

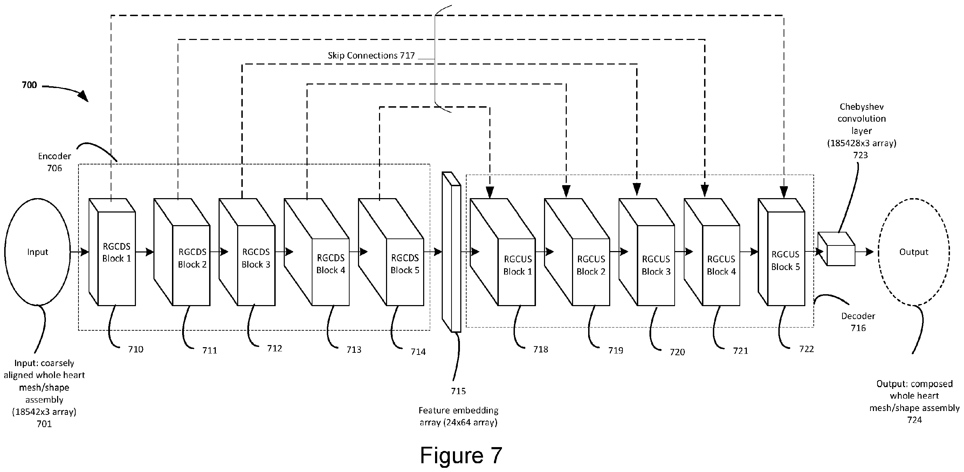

As shown in Figure 1, the disclosed spatial composition module 120 applies two networks, namely, 1) an affine composition network (see Figure 6) and 2) a non-rigid composition network (see Figure 7). The affine composition network first recovers affine transformations to coarsely compose the input anatomical parts'/organs' shapes. The coarsely composed parts are in turn used as inputs to the non-rigid composition network, which estimates localised non-rigid/deformable transformations, and outputs finely composed multi-part/multi-organ shape assemblies, i.e. virtual chimeras. The affine composition network comprises N branches/sub-networks, where each sub-network takes one of N anatomical part/organ's shape as input (where N is determined by the structures of interest in the multi-part/multi-organ shape assemblies to be synthesised). In the present example, the affine composition network comprises N=5 part-specific sub-networks corresponding to the aforementioned five cardiac structures of interest. The non-rigid composition network, meanwhile, comprises a single network that takes the coarsely composed multi-part/multi-organ shape assembly output by the affine composition network as input. The affine composition network and non-rigid composition network are trained separately, and one after the other (i.e. sequentially, so that the affine network is trained first, followed by the non-rigid network) in the present example, but may also be trained jointly. Both the affine composition network and the non-rigid composition network may be trained with real data or synthetic data or a combination of the two, i.e. they may be trained with input shapes derived from real patient data or using shapes synthesised by a generative network such as the independent generator 200 or dependent generator 300 disclosed above (or some combination of real and synthetic data). As such, the overall spatial composition network applied by the spatial composition module 120 is not restricted to using real or synthetic shapes for training, and, additionally, is not restricted to using synthetic data generated by the disclosed independent 200 and dependent 300 generators either. In the present example, a combination of real and synthetic data was used to train both the affine composition and non-rigid composition networks of the spatial composition module 120.

Given meshes for all N parts in the shape assembly of interest, denoted, $\{\mathbf{x}\}^{n=1\ldots N}$ (where the N parts' meshes may be derived from real patient data or may be synthesised using a generative shape model, e.g. independent 200 or dependent 300 generator, or may be a combination of real and synthetic data), each of the N parts is passed to its own specific branch/sub-network in the affine composition network as input. In the present example, shapes for the left ventricle, right ventricle, left atrium, right atrium and aortic root are passed to their specific branch/sub-network (A-E) as inputs. Each sub-network in the affine composition network extracts information regarding the position, orientation and shape of its corresponding input mesh and outputs 3D affine transformations for each part in the shape assembly, resulting in N affine transformations, denoted $\{\mathbf{T}\}^{n=1\ldots N}$. In the present example, five affine transformations are estimated by the affine composition network, corresponding to the five input cardiac structures of interest (LV, RV, LA, RA, AR). Each of the five (N=5) affine transformations estimated comprise eight parameters, including translation (denoted $\mathbf{t}$ = $[t_x, t_y, t_z]$, where each component in the translation vector $\mathbf{t}$ represents the estimated displacement along a specific direction/axis in 3D Euclidean space), rotation (denoted $\mathbf{R}$ = $[Q_1, Q_2, Q_3, Q_4]$, where the components Q1-4 represent quarternions used to parameterise rotations in 3D) and scaling (denoted S, which is a scalar value that controls the global scaling estimated for each part). The affine composition network is trained in a 'self-supervised manner' to estimate affine transformations for each of the five cardiac structures of interest by minimising a loss function $L_{affine}$ given by:

$$L_{affine} = \sum_{n=1}^{N} || v_o^{transf} - T^n v_n ||_1 \quad (9)$$

Here, $\mathbf{T^n}$ represents the 3D affine transformation estimated and applied to part $\mathbf{x}_n$, where subscript {n=1...N} represents each part of interest in the shape assembly (in the present example N=5, corresponding to the five cardiac structures of interest); $\mathbf{v}_n$ represents the nodes/vertices in the mesh of the n$^{th}$ part in the assembly, i.e. $\mathbf{x}_n$, which are shared with all other (N-1) parts in the assembly; $\mathbf{v}_o^{transf}$ represents the nodes/vertices in the meshes of all other (N-1) parts in the shape assembly that are shared with the mesh of the n$^{th}$ part. $\mathbf{v}_o^{transf}$ is constructed by concatenating shared vertices from meshes of all (N-1) parts in the assembly (given part $\mathbf{x}_n$), following application of the affine transformations estimated for each of the (N-1) parts, i.e. $v_o^{transf} = \{T^m v_m\}^{m=(1...N-1)}$, where, $\mathbf{v}_m$ denotes the vertices shared between part 'm' in the assembly and part 'n', for m≠n and $\mathbf{T^m}$ denotes affine transformations estimated for the m$^{th}$ part in the assembly. Because the training of the affine composition network is driven by a loss function that depends only on the shared nodes/vertices between adjacent/neighbouring parts in the assembly, the network is said to be trained in a 'self-supervised' manner. This is in contrast with fully supervised learning where ground truth affine transformations must be known a priori (i.e. beforehand) to drive the training of the affine composition network.

Figure 6 shows an example architecture for the affine composition network according to the present disclosure. In brief summary, a respective input mesh 601 (e.g. 601A for the LV, etc) is inputted to a respective encoder sub-network 606, which operates to down-sample the data, and output a respective fully connected layer Latent vector 615. This output Latent vector 615 is then concatenated into a shared latent vector 616, followed by two fully connected layers - layer 1 617 and layer 2 618. Fully connected layers, also known as dense layer, and multi-layer perceptrons are stacked individual neurons/neural units where each neuron in a fully connected layer is connected to every feature of the vector input to the layer. The second fully connected layer 618 provides the input into each of three sub-branches that handle one of the three transformations - i.e. scaling, rotation and translation. These are handled by two fully connected layers (619/620, 622/623, 625/626) for each, which output a respective set of parameters as a vector - scaling output vector 621, rotation output vector 624, translation output vector 627, Here the fully connected layers which output the translation, rotation and scaling parameters for all five cardiac structures of interest, act as regression models/networks. Details of the five branches/sub-networks that may be used in an example of the affine composition network are presented in Table 7. Each branch/sub-network is made up of five residual graph convolution down-sampling blocks (as shown in Figure 4). The number of feature channels used within each graph convolution layer in the residual graph convolution down-sampling blocks, from blocks 1 to 5 are: 16, 32, 32, 64 and 64. The mesh down-sampling factors used across down-sampling blocks 1 to 5 were different for each branch/sub-network in the affine composition network. For each part-specific branch/sub-network, the down-sampling factors used according to an example are as follows - left ventricle branch/sub-network: [4,4,4,6,6]; right ventricle branch/sub-network: [4,4,4,6,6]; left atrium branch/sub-network: [4,4,4,5,5]; right atrium branch/sub-network: [4,4,4,5,5]; and aortic root branch/sub-network: [4,4,4,4,4], where each number within brackets corresponds to the down-sampling factor used in residual graph convolution down-sampling blocks from block 1 to 5. The outputs of the final residual graph convolution down-sampling blocks in each branch/sub-network is flattened to a vector. The resulting flattened vectors from each branch/sub-network are subsequently concatenated into a single long vector which is passed as input to a sequence of two fully connected layers. These two fully connected layers extract features from the provided input and pass the output in the form of a shorter vector (one divided by 4 in the present example) to three regression branches comprising two fully connected layers each. These three regression branches in turn estimate the translation, rotation and scaling parameters (which represent the desired 3D affine transformations) for all parts in the assembly.

Table 7: An example network architecture that may be used for an affine composition network in the spatial composition module 120 (comprising five part-specific branches/sub-networks corresponding to the five cardiac structures of interest).

| Branch-1 | Branch-2 | Branch-3 | Branch-4 | Branch-5 |
|---|---|---|---|---|
| Input: LV mesh/ unstructured graph ($x_i$), size: (VLVX 3) | Input: RV mesh/ unstructured graph ($x_i$), size: (VRVX 3) | Input: LA mesh/ unstructured graph ($x_i$), size: (VLAX 3) | Input: RA mesh/ unstructured graph ($x_i$), size: ($V_{RA} \times$ 3) | Input: AR mesh/ unstructured graph ($x_i$), size: ($V_{AR} \times$ 3) |
| Residual Graph Convolution Down-sampling Blocks 1-5 | Residual Graph Convolution Down sampling Blocks 1-5 | Residual Graph Convolution Down sampling Blocks 1-5 | Residual Graph Convolution Down sampling Blocks 1-5 | Residual Graph Convolution Down sampling Blocks 1-5 |
| Flatten layer, size: (1472X1) vector | Flatten layer; size: (1280X1) vector | Flatten layer; size: (1408X1) vector | Flatten layer; size: (1280X1) vector | Flatten layer; size: (1280X1) vector |
| Concatenate Layer combining outputs from all part-specific branches/sub-networks into a shared representation, size: (6720x1) vector | | | | |
| Two Fully connected layers; output size: (1680x1) vector | | | | |
| Translation regression layers (two fully connected layers), output size: (15x1) vector; comprising translation vectors (3 parameters each) specific to each of the five cardiac structures | Rotation regression layers (two fully connected layers), output size (20x1) vector; comprising rotation vectors (4 parameters each) specific to each of the five cardiac structures | | Scaling regression layers (two fully connected layers), output size (5x1) scalar value; comprising global scaling values (scalar value) specific to each of the five cardiac structures | |
| Outputs: 3D affine transformation for LV; denoted $T_{LV}$ comprising 8 parameters (i.e. 3 translation parameters, 4 rotation parameters, and 1 scaling parameter) | Outputs: 3D affine transformation for LV; denoted $T_{LV}$ comprising 8 parameters (i.e. 3 translation parameters, 4 rotation parameters, and 1 scaling parameter) | Outputs: 3D affine transformation for LV; denoted $T_{LV}$ comprising 8 parameters (i.e. 3 translation parameters, 4 rotation parameters, and 1 scaling parameter) | Outputs: 3D affine transformation for LV; denoted $T_{LV}$ comprising 8 parameters (i.e. 3 translation parameters, 4 rotation parameters, and 1 scaling parameter) | Outputs: 3D affine transformation for LV; denoted $T_{LV}$ comprising 8 parameters (i.e. 3 translation parameters, 4 rotation parameters, and 1 scaling parameter) |

Following affine spatial composition of real and/or synthetic parts using the affine composition network of Figure 6, the coarsely composed/aligned multi-part shape assemblies, denoted $X = \{x_n\}^{n=(1...N)}$, may be input to a non-rigid composition network for refinement. Here subscript 'n' is used to represent each of the N parts in the multi-part shape assembly (i.e. N=5 in the current example, corresponding to the five cardiac structures of interest). The non-rigid composition network estimates localised part-wise deformations to improve continuity or coherence at boundaries between adjacent parts in a multi-part shape assembly, by reducing any intersections/gaps between adjacent parts that remain following the initial affine composition step. The non-rigid composition network is a graph-convolutional neural network with an encoder-decoder network pair (depicted in Figure 7), similar to the gcVAEs 202A-E in the independent generator 200 of Figure 2. This non-rigid composition network 700 extracts features through a series of graph convolution blocks (710-714) from the coarsely composed/aligned multi-part shape assemblies (i.e. whole heart shape assemblies in the current example) and estimates localised node-/vertex-wise displacements in meshes 701 of each part in the assembly. The non-rigid composition network 700 is trained in a self-supervised manner (as with the affine registration network) by minimising a loss function $L_{(non-rigid)}$ given by:

$$L_{non-rigid} = \sum_{n=1}^{N} || v_o^{nr} - T_n^{nr} v_n ||_1 + w_3 L_{laplacian} + w_4 || T^{nr} ||_1 \quad (10)$$

Here, $\mathbf{T}^{nr}$ represents the non-rigid transformations, i.e. the node-/vertex-wise displacements, estimated for all vertices in meshes of all parts $\{\mathbf{x}_n\}^{n=(1...N)}$ in the coarsely aligned multi-part shape assembly $\mathbf{X}$ output from the preceding affine composition step; $\mathbf{T}_n^{nr}$ represents the displacements estimated for the nodes/vertices shared between the mesh of the 'nth' part in the assembly (denoted $\mathbf{v}_n$), and the meshes of all other (N-1) parts in the assembly; $\mathbf{v}_o^{nr}$ represents the deformed/transformed nodes/vertices in the meshes of all other (N-1) parts in the shape assembly that are shared with the mesh of the nth part. $\mathbf{v}_o^{nr}$ is constructed by concatenating shared vertices from meshes of all (N-1) parts in the assembly (given part $\mathbf{x}_n$), following application of the node-/vertex-wise displacements estimated for each of the (N-1) parts, i.e. $v_o^{nr} = \{T_m^{nr} v_m\}^{m=(1...N-1)}$, where, $\mathbf{v}_m$ denotes the vertices shared between part 'm' in the assembly and part 'n', for $m \neq n$ and $\mathbf{T}_m^{nr}$ denotes non-rigid/deformable transformation estimated for the $m^{th}$ part in the assembly. Because the training of the non-rigid composition network is driven by a loss function that depends only on the shared nodes/vertices between adjacent/neighbouring parts in the assembly, the network is said to be trained in a 'self-supervised' manner. This is in contrast with fully supervised learning where ground truth non-rigid transformations must be known a priori (i.e. beforehand) to drive the training of the affine composition network. The first term (i.e. $||v_o^{nr} - T_n^{nr} v_n||_1$) in $L_{(non-rigid)}$ is designed to minimise the distance between nodes/vertices that are shared across each pair of adjacent structures in the assembly, by iteratively refining estimates for $\{T_n^{nr}\}^{n=(1...N)}$ as the training progresses for the non-rigid composition network. The other terms in $L_{(non-rigid)}$, namely, $L_{laplacian}$ and $||\mathbf{T}^{nr}||_1$ are designed to encourage the estimated non-rigid transformations to be smooth and sparse/localised (i.e. meaning pushing lots of displacements/transformations to be equal to zero where no deformation is desired, and enabling the deformation to be localised to the region of shared vertices only - i.e. for displacements to be non-zero at shared vertices), respectively. $L_{laplacian}$ is defined exactly the same as previously in the loss function used to train the independent and dependent generators (refer to equation 4 above). The third term in $L_{(non-rigid)}$ represents the Li-norm applied to the displacements estimated for all nodes/vertices of all parts in the assembly, and is used to encourage sparsity such that the estimated deformations/non-rigid transformations are localised to boundaries between adjacent structures in the assembly (i.e. to nodes/vertices shared between parts in the assembly). The weights $w_3$ and $w_4$ are used to balance the relative influence of the second and third loss terms, respectively, with the first term in $L_{(non-rigid)}$, and are hyperparameters that are tuned empirically.

Details of the encoder-decoder network pair that may be used in the non-rigid composition network are presented in Table 8. The encoder 706 and decoder 716 networks comprise five blocks each, where each block learns shape features at a different spatial resolution, enabling the overall non-rigid composition network 700 to learn shape features with both global and local context, across multiple mesh resolutions. This is enabled by including mesh down-sampling and up-sampling operations in each of the five blocks (710-714) of the encoder 706 and the five blocks (718-722) of the decoder 716 networks respectively. The encoder 706 takes as input the coarsely composed/aligned multi-part shape assembly, which in the case of the current example is the whole-heart shape assembly comprising meshes for the left ventricle (LV), right ventricle (RV), left atrium (LA), right atrium (RA) and aortic root (AR). The features extracted through the residual graph convolution down-sampling blocks (710-714) in the encoder network are output to a feature embedding array 715 and the residual graph convolution up-sampling blocks (718-722) in the decoder network are used to unravel/expand the feature embedding array 715. The output of the final residual graph convolution up-sampling block is condensed to the size of the input array/coarsely aligned whole heart mesh using a Chebyshev convolution layer 723 and the Chebyshev convolution layer 723 is used to estimate node-/vertex-wise displacements for all parts in the assembly, which is output 724 by the decoder. The residual graph convolution down-sampling blocks and up-sampling blocks used in the encoder and decoder, respectively, are depicted in Figure 4. The number of feature channels used within each graph convolution layer in the residual graph convolution down-sampling blocks, from blocks 1 to 5 are: 16, 32, 32, 64 and 64, respectively. Similarly, the number of feature channels used within each graph convolution layer in the residual graph convolution up-sampling blocks, from blocks 1 to 5 are: 64, 64, 32, 32, and 16, respectively. Skip connections 717 are included between each corresponding encoder 706 and decoder 716 block within the network, to ensure better propagation of features and gradients during training. The mesh down-sampling factors used across down-sampling blocks 1 to 5 were [4,4,4,6,6], where, each number within brackets corresponds to the down-sampling factor used in residual graph convolution down-sampling blocks from block 1 to 5. Similarly, the up-sampling factors used across up-sampling bocks 1 to 5 were [6,6,4,4,4], where each number within brackets corresponds to the up-sampling factor used in residual graph convolution up-sampling blocks from block 1 to 5. The output of the final residual graph convolution up-sampling block in the decoder is passed to a single convolution layer which estimates the node-/vertex-wise displacements for all parts, resulting in the final composed whole-heart shape assembly, i.e. a cardiac virtual chimera.

Table 8: An example network architecture for use in a non-rigid composition network in the spatial composition module 120.

| Encoder | Decoder |
|---|---|
| Input: Coarsely aligned/composed whole-heart shape assembly comprising meshes/unstructured graphs for all N=5 parts, size: (185428 $\times$ 3) | Input: Feature embedding, size: (24x64) array |
| Residual Graph Convolution Down-sampling Blocks 1-5 | Residual Graph Convolution Up sampling Blocks 1-5 |
| Output: Feature embedding, size: (24x64) array | Chebyshev convolution layer, size: (185428 $\times$ 3) |
| | Output: Node-/vertex-wise displacements for all parts whole-heart shape assembly |

Data used for training overall generative shape compositional framework:
The disclosed generative shape modelling system, also known as a generative shape compositional framework, for synthesising organ virtual chimera (e.g. in the specific example, cardiac virtual chimera that describe virtual whole-heart shape assemblies) cohorts may be trained and evaluated using data derived from real individuals' data, for example as available in the UK Biobank imaging database. The general data requirements and the specific data and pre-processing steps employed in the current example, for training the disclosed generative shape compositional framework include -

All anatomical parts' and/or organs' shapes are represented as surface or volumetric meshes/unstructured graphs but may also be represented as contours in the form of an unstructured graph. In the current example all cardiac structures of interest (i.e. left ventricle, right ventricle, left atrium, right atrium and aortic root) were represented as triangular surface meshes, but other forms may equally be used (e.g. polygonal meshes).

The mesh/unstructured-graph based representations of all parts' and/or organs' shapes used in the training population are to have the same number of nodes and mesh connectivity/graph topology, prior to their use for training the disclosed generative framework. This can be achieved by co-registering all samples of a specific part to establish spatial correspondences across the training population considered for that part, i.e. for example all left ventricle meshes considered in the training population are co-registered to one another, while all right ventricle meshes are co-registered to each other and so on. The training populations for each part can vary in size, and the individuals/patients whose data are used in the part-specific training populations can also vary in terms of degree of individual/patient overlap across different parts' training populations.

According to an example, a high-resolution 3D cardiac atlas mesh (where atlas refers to an average representation of a population of cardiac meshes derived from multiple patients' images) available from a previous study may be non-rigidly registered to contours defining the boundaries of the cardiac chambers annotated manually for each of the four cardiac chambers of interest, in cardiac cinematic magnetic resonance (cine-MR) images by expert cardiologists. These manual contours are available in the UK Biobank database. By registering the high-resolution 3D cardiac atlas mesh to the manual contours, the training populations of shapes for each part/cardiac structure of interest may be generated (i.e. as the same atlas mesh was deformed/non-rigidly registered to fit the manual contours of all individuals considered from UK Biobank, spatial correspondence was automatically established across all samples in all part-specific training populations). The high-resolution cardiac atlas used to generate the training populations comprised the following parts/structures (each represented by distinct meshes) as part of a multi-part shape assembly - left and right ventricles (LV and RV), left and right atria (LA and RA) and the aortic vessel root (AR). Additionally, the atlas comprised shared vertices between adjacent structures' meshes (i.e. at the boundaries separating any pair of adjacent structures in the whole-heart shape assembly). Vertices in the atlas mesh were shared at boundaries between the following pairs of structures - LV-RV, LV-LA, LA-RA, RV-RA, LV-AR, LA-AR, and RA-AR. The availability of these shared vertices between adjacent parts/structures helps enable the self-supervised learning scheme used to train the disclosed spatial composition network (i.e. both the affine and non-rigid spatial composition networks). The disclosed formulation of the generative shape compositional framework thus uses information regarding shared vertices between adjacent parts in the multi-part and/or multi-organ shape assemblies, where the shared vertices information may be created manually/semi-automatically/automatically, or be available prior to training the spatial composition network in the framework. Although the manual contours, and hence the generated training populations of part-specific meshes (based on the deformed/registered atlas mesh), were defined on cardiac cine-MR images of individuals in the UK Biobank, the disclosed approach is not dependent on the part-specific training populations of meshes being extracted/inferred from cardiac cine-MR images only. Any part and/or organ shape of interest may be obtained from different imaging modalities and also from different patient populations. For example, the left ventricular meshes may be extracted/inferred from cardiac cine-MR images of patients/individuals in population 'A', while the aortic root meshes may be extracted/inferred from cardiac computed tomography angiography (CTA) images of patients/individuals in population 'B'. Additionally, the patients/individuals included in populations 'A' and 'B' may be - (a) non-overlapping; (b) partially-overlapping; or (c) completely-overlapping; where, scenario (a) means that no patient in population 'A' is included in population 'B'; scenario (b) means that some proportion of patients in population 'A' are included in population 'B'; and scenario (c) means that all patients included in population 'A' are included in population 'B', and vice versa. It is important to note however, that for scenario (a) where, the training populations for each individual part/structure are extracted/inferred from images of completely different patients/patient populations, only the independent generator 200 within the disclosed generative shape compositional framework is suitable to be used, i.e. while the independent generator is designed to allow training with non-overlapping data (and partially-overlapping and completely-overlapping data), the dependent generator can only be trained with either partially-overlapping or completely-overlapping data.

According to an example, the overall cohort used to train and test the disclosed generative shape compositional framework comprised 2360 subject-specific whole-heart shape assemblies (represented as meshes). These subject-specific meshes were created by registering the high-resolution cardiac atlas mesh to the manually annotated cardiac contours available for individuals in the UK Biobank database. These 2360 subjects were selected from 4000 subjects in the UK Biobank for whom the manually annotated cardiac contours are available. This selection was done based on visual assessment of the quality of the subject-specific meshes resulting from the registration process (initially executed for all 4000 subjects). Only those meshes were retained where no obvious topological errors were introduced following registration of the atlas mesh to the manual contours, resulting in the chosen cohort of 2360 subject-specific meshes. The resulting cohort of 2360 subject-specific meshes shared node/vertex-wise spatial correspondence across all parts/cardiac structures in the shape assembly, i.e. all meshes have the same number of vertices/nodes, the same mesh connectivity/graph topology, and each node/vertex across all meshes represent the same anatomical feature/position. Having the same mesh connectivity/unstructured graph topology means that the edges that connect each node to neighbouring nodes in the mesh/unstructured graph are identical across all subject-specific meshes in the cohort. Another way to define having the same mesh connectivity/unstructured graph topology is to say that the adjacency matrices of all subject-specific meshes/unstructured graphs were identical. Identical graph topology across all subject-specific meshes enables the use of spectral (i.e. Chebyshev polynomial-based) convolutions in the graph-convolution layers used throughout the disclosed generative shape compositional framework, as fixed/identical graph topology across all inputs is a pre-requisite for graph neural networks that utilise spectral convolution operations in their constituent graph convolution layers. The developed system may be adapted to work with meshes that are not identical in terms of their vertex connectivity/graph topology by utilising spatial graph convolution operations in place of spectral convolution operations. The cohort of 2360 subject-specific meshes/whole-heart shape assemblies were randomly split into training, validation and test sets, each comprising 422, 59, and 1879 samples, respectively. An aim of the split may be to have a substantially larger test set than training set to ensure evaluation is on a more diverse population than used for training.

The disclosed generative shape compositional framework may be trained with non-overlapping, partially-overlapping or completely-overlapping data as defined previously. However, in the current example, the training of the disclosed generative shape compositional framework was carried out using partially-overlapping and completely-overlapping data derived from cardiac cine-MR images available in the UK Biobank. Only the partially-overlapping and completely-overlapping scenarios were implemented for fair comparison between the disclosed independent and dependent generators (as the latter cannot be trained with non-overlapping data, unlike the independent generator). For the completely-overlapping scenario, all subjects' data included in the training set contain all cardiac structures/parts in the whole-heart shape assembly (i.e. LV, RV, LA, RA and AR), and the complete data set comprising 422 training samples (with five parts/cardiac structures each) was used to train both the independent and dependent generators. For the partially-overlapping scenario, 300 subjects in the training set were considered to have 'missing data' by including just one part/cardiac structure from each subject in the training set (i.e. this was done to mimic a real scenario where only partially overlapping data across subjects is available). The samples in the training set for the partially-overlapping scenario included 60 parts/cardiac structures for each of the five cardiac structures of interest, obtained from different subjects (resulting in 300 training samples, each of a different part/cardiac structure obtained from different subjects). The remaining 122 subjects in the training set were considered to have complete whole-heart shape assemblies (i.e. all five parts/cardiac structures were available). This partially-overlapping data set was also used to train both the independent and dependent generators.

The spatial composition network (comprising both the affine and non-rigid spatial composition networks) was trained using both real and synthetic data. The real data was used first to pre-train the spatial composition network, keeping the same data splits for training, validation and testing as used for the part-aware generative module trained in the 'completely-overlapping' scenario (i.e. 422 samples used for training, 59 samples used for validation and 1879 samples used for testing). Following initial training using real data, the spatial composition network was fine-tuned (i.e. training was continued) using synthetic data generated using the independent generator 200 (i.e. using parts/structures synthesised using each part-specific gcVAE in the independent generator 200). A total of 2000 synthetic samples for each part/cardiac structure of interest was included in the synthetic data cohort. Among these 1600 were used for training, 200 for validation and 200 for testing.

Figure 8 shows a method of training a part-aware generative model 800, according to an example of the disclosure. The method starts at 801 by using certain non-overlapping or partially-overlapping or complete-overlapping anatomical shape/mesh data (from multiple subjects/patients) as inputs (i.e. training data) to the part-aware generative model 810. The part-aware generative model is trained 810 to learn a hidden/latent representation of the inputted data by minimising a suitable loss function through numerical optimisation and by using the error backpropagation algorithm to learn/update the parameters of the part-aware generative model. The training process is iterative wherein, the error/loss incurred by the part-aware generative model at each iteration (also referred to as epoch) is used to update the parameters of the part-aware generative model at the end of each iteration/epoch. Training continues until a suitable convergence criterion is reached, or a user-specified maximum number of training iterations/epochs 820 is reached, at which point, the training loop is stopped/terminated 830 and the learned parameters of part-aware generative model are saved.

Figure 9 shows a method of training a spatial composition model 900, according to an example of the disclosure. The method starts at 901 by using training data wherein, each training sample comprises all parts in the shape assembly of interest as inputs. The constituent parts in each training sample used to start training 910 the spatial composition model may belong to real patients/subjects, or may be synthetic/virtual parts synthesised using a suitable statistical/machine learning model (e.g. either the independent generator 200 or the dependent generator 300 in the disclosed system). The spatial compositional model is trained 910 to learn the spatial relationships between the inputted parts of the shape assembly and to estimate the spatial transformations necessary to compose/align (i.e. put together) the parts into anatomically plausible/realistic shape assemblies. The training process is iterative and involves minimisation of a suitable loss function through numerical optimisation and by using the error backpropagation algorithm to learn/update the parameters of the spatial composition model. During the iterative training process, the error/loss incurred by the spatial composition model at each iteration (also referred to as epoch) is used to update the parameters of the spatial composition model at the end of each iteration/epoch. Training continues until a suitable convergence criterion is reached, or a user-specified maximum number of training iterations/epochs 920 is reached, at which point, the training loop is stopped/terminated 930 and the learned parameters of spatial composition model are saved.

Whilst Figures 8 and 9 show the training being done independently, the training may also be carried out sequentially, i.e. training the part-aware model then the spatial composition model for one cycle and repeating that for the multiple epochs.

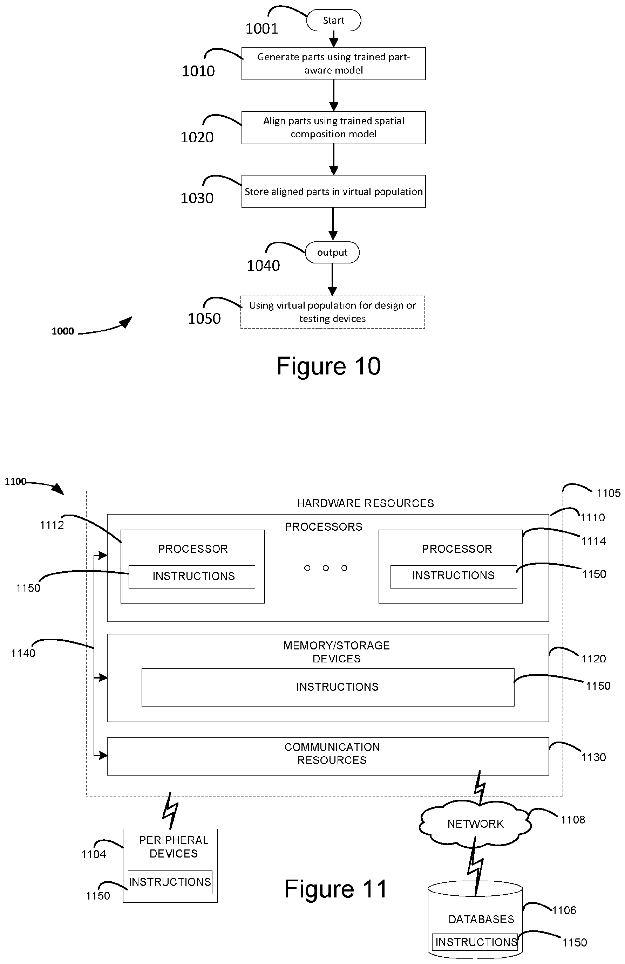

Figure 10 shows a method of generating a virtual chimera population 1000 for use in in-silico trials, according to an example of the disclosure. According to this example, which may be a computer-implemented method where the organ shapes may be represented as surface or volumetric meshes, the method starts at step 1001, and then comprises the steps of: 1010 - generating organ parts using, for example, the part-aware generative model that has been trained on non-overlapping or partially-overlapping (or completely-overlapping) patient data (in order to learn a latent representation of each part of a multi-part organ shape for inclusion in a virtual population) and arranged to output synthesised parts of the multi-part organ shape; 1020 aligning the previously outputted synthesised parts using, for example, the spatial composition model that has also been trained on non-and partially-overlapping real patient data or synthetic/virtual patient data (to align the outputted synthesised parts of the multi-part organ shape) and output an anatomically meaningful example of an overall multi-part organ shape as a virtual chimera; and 1030- storing the resultant virtual chimera in a combined set of chimeras, in a so-called the virtual chimera population. The resultant complete virtual chimera population may then be outputted 1040, for multiple subsequent uses - for example, for use in designing or testing medical devices, in particularly for use in an in-silico trial.

Generating virtual populations of anatomy and physiology as disclosed herein is important for in-silico trials because it provides a systematic and exhaustive framework for investigating and evaluating the performance of the medical devices in-silico. A virtual population may be a collection of samples (for example called virtual patients) that represent plausible instances of anatomy and/or physiology that could realistically be found in a target patient population for the medical device under test, but which are not necessarily representative of any real patients' data. Virtual populations may be synthesised as disclosed herein using statistical or machine learning approaches, where the machine learning approaches are initially trained on real patients' data to learn the underlying hidden (or latent) distribution of the data. The trained statistical/machine learning models may then be used to create new data, i.e. new virtual patients, by sampling from the learned latent distributions. The resulting virtual population created by sampling from such trained statistical/machine learning models may be referred to as a virtual population of anatomy and/or physiology (depending on the type of real patients' data used to train the statistical/machine learning model).

Existing approaches to synthesising virtual patient populations require complete overlap in the data used to build the models, therefore limiting their ability to exploit large disparate datasets, particularly with non-overlapping or partially-overlapping information. The inability to leverage non-/partially-overlapping data (and thereby maximise their value) also limits the overall anatomical and physiological variability that is captured in virtual patient populations synthesised using existing approaches.

The virtual chimera/models that are outputted by the disclosed system are complete, and anatomically realistic, models of the physical construction of the respective organ, or sets of organs, and as such may be used for any purpose utilising such models, in particular in-silico trials of medical devices for purposes of development, design, and manufacture of medical devices, and the subsequent medical trials thereof. For example, the cardiac specific virtual chimera populations generated/created using the disclosed system may be used for conducting in-silico trials of all implantable cardiac devices such as - transcatheter aortic valve implantation devices, mitral valve replacement devices, cardioverter defibrillators, pacemakers, and coronary stents. In-silico studies investigating the effects of drugs, genetic, lifestyle, environmental or demographic factors on the biochemical and/or biophysical processes in the heart through computational simulation and modelling may also find use for the virtual populations generated using the disclosed system. The generated virtual populations may also be used for conducting virtual imaging trials and developing and calibrating non-implantable medical devices such as: medical imaging systems (e.g. intravascular ultrasound, computed tomography, computed tomography angiography, magnetic resonance imaging, positron emission tomography, single-photon emission computed tomography, cardiac ultrasound/echocardiography, and 3D ultrasound); interventional robots; surgical guidance systems; catheters, guidewires, etc. The generated virtual populations may also be used for 3D printing realistic physical cardiac models/phantoms for use in experimental studies, developing surgical guidance systems, surgical training and educational purposes.

As may now be appreciated, some methodologies for providing virtual chimera use 'strong labels' within a self-supervised or fully supervised learning framework for training the neural networks used to create the virtual chimera. 'Strong labels' refer to reference/ground truth spatial transformations, and rely on the real and complete multi-part/ shape assemblies being available. As such, with availability of the reference/ground truth spatial transformations, the neural networks may be trained in a fully supervised manner to predict the spatial transformations necessary to compose the individual parts into complete multi-part shape assemblies. Getting access to the reference/ground truth spatial transformations requires prior availability/knowledge of the corresponding real and complete multi-part shape assemblies. However, training the neural network using Strong labels precludes the use of partially-overlapping (or even non-overlapping) data. Accordingly, the present disclosure provides a novel self-supervised approach, trainable with weak labels, for the composition network, where weak labels refer to information that is shared between adjacent parts in real and incomplete shape assemblies (i.e. comprise partially-overlapping or non-overlapping data).

Examples of the present disclosure may be implemented by suitably programmed computer hardware. Fig. 11 is a block diagram 1100 illustrating components, according to some example embodiments, able to read instructions from a machine-readable or computer-readable medium (e.g., a non-transitory machine-readable storage medium) and perform any one or more of the methodologies discussed herein, hence providing the apparatus or system to carry out the described virtual population modelling. Specifically, Fig. 11 shows a diagrammatic representation of hardware resources 1105 including one or more processors (or processor cores) 1110, one or more memory/storage devices 1120, and one or more communication resources 1130, each of which may be communicatively coupled via a bus 1140. The processors 1110 (e.g., a central processing unit (CPU), a reduced instruction set computing (RISC) processor, a complex instruction set computing (CISC) processor, a graphics processing unit (GPU), a digital signal processor (DSP) such as a baseband processor, an application specific integrated circuit (ASIC), a cloud processing function (such as AWS instance), another processor, or any suitable combination thereof) may include, for example, a processor 1112 and a processor 1114.

The memory/storage devices 1120 may include main memory, disk storage, or any suitable combination thereof. The memory/storage devices 1120 may include, but are not limited to any type of volatile or non-volatile memory such as dynamic random access memory (DRAM), static random-access memory (SRAM), erasable programmable read-only memory (EPROM), electrically erasable programmable read-only memory (EEPROM), Flash memory, solid-state storage device (SSD), magnetic storage based hard disk drive (HDD) media, etc.

The communication resources 1130 may include interconnection or network interface components or other suitable devices to communicate with one or more peripheral devices 1104 or one or more databases 1106 via a network 1108. For example, the communication resources 1130 may include wired communication components (e.g., for coupling via Ethernet, a Universal Serial Bus (USB) or the like), cellular communication components, NFC components, Bluetooth® components (e.g., Bluetooth® Low Energy), Wi-Fi® components, and other communication components.

Instructions 1150 may comprise software, a program, an application, an applet, an app, or other executable code for causing at least any of the processors 1110 to perform any one or more of the methodologies discussed herein. The instructions 1150 may reside, completely or partially, within at least one of the processors 1110 (e.g., within the processor's cache memory), the memory/storage devices 1120, or any suitable combination thereof. Furthermore, any portion of the instructions 1150 may be transferred to the hardware resources 1105 from any combination of the peripheral devices 1104 or the databases 1106. Accordingly, the memory of processors 1110, the memory/storage devices 1120, the peripheral devices 1104, and the databases 1106 are examples of computer-readable and machine-readable media.

In some embodiments, the electronic device(s), network(s), system(s), chip(s) or component(s), or portions or implementations thereof, of Figures 11, or some other figure herein may be configured to perform one or more processes, techniques, or methods as described herein, or portions thereof.

In a particular example the disclosed generative shape compositional framework may be implemented using PyTorch, PyTorch Geometric and PyTorch3D python libraries on a PC with an NVIDIA RTX 2080Ti graphics processing unit (GPU).

In an example, the part-aware generative module (i.e. both the independent and dependent generators) were trained using the Adam optimizer with an initial learning rate of $5e^{-04}$ and a learning rate decay of 0.9 per epoch of training. All networks in the part-aware generative module were trained with a batch size of 16, for a maximum of 100 training epochs. The affine and non-rigid spatial composition networks were both trained with a batch size of 8 and for a maximum of 100 training epochs. The initial learning rate used for both spatial composition networks was $1e^{-04}$ with a learning rate decay of 0.9 per epoch. All hyperparameters associated with the networks' architectures, training routines, and the optimiser used were tuned/set empirically based on the average loss values observed for the validation set in preliminary prototyping experiments.

While training both the independent 200 and dependent 300 generator, a warm-up strategy may be adopted to improve stability and prevent mode collapse in the learned posterior distribution over the latent variables. This is achieved by initially training the part-wise gcVAEs in the independent generator, and the gcmcVAE in the dependent generator, with the weight of KL loss term (i.e. $\omega 0$ in equation 1) set to zero for 200 epochs (i.e. they are trained as plain autoencoders). The learned weights initialise the subsequent training step for the independent and dependent generator, wherein, the weight of the KL loss term is initially set to a small value, i.e. 1e-6, and subsequently increased each epoch, for example by multiplying by a factor of 1.25, up to a maximum value of 1e-4. The other weights in the regularisation terms of the composite loss function (refer to equation 3) and that of self-supervised registration loss function, namely, $\omega 1$, $\omega 2$, $\omega 3$ and $\omega 4$ were set to 8, 1, 8, and 5e-7, respectively.

The same parameter setup of the network architecture and optimisation as the part-aware generator may be retained for the composition network, except for setting the initial learning rate to 1e-4 and batch size to 8. The part shapes may be randomly sampled from different patients data during training of the composition network. The composition network may be pre-trained with the original shapes in the real population for, e.g., 100 epochs, and then it may be trained on previously generated shapes. The generated dataset may be randomly split into portions for each of training, validation, and testing (e.g. 1600/200/200 split). Notably, the composition network is trained with only partially overlapping data, i.e., structures are obtained from different patients.

In the foregoing, functions are described as modules or blocks, i.e. functional units that are operable to carry out the described function, algorithm, or the like. These terms may be interchangeable. Where modules, blocks, or functional units have been described, they may be formed as processing circuitry, where the circuitry may be general purpose processor circuitry configured by program code to perform specified processing functions. The circuitry may also be configured by modification to the processing hardware. Configuration of the circuitry to perform a specified functions may be entirely in hardware, entirely in software or using a combination of hardware modification and software execution. Program instructions may be used to configure logic gates of general purpose or special-purpose processor circuitry to perform a processing function.

Circuitry may be implemented, for example, as a hardware circuit comprising custom Very Large Scale Integrated, VLSI, circuits or gate arrays, off-the-shelf semiconductors such as logic chips, transistors, or other discrete components. Circuitry may also be implemented in programmable hardware devices such as field programmable gate arrays, FPGA, programmable array logic, programmable logic devices, A System on Chip, SoC, or the like.

Machine readable program instructions may be provided on a transitory medium such as a transmission medium or on a non-transitory medium such as a storage medium. Such machine readable instructions (computer program code) may be implemented in a high level procedural or object oriented programming language. However, the program(s) may be implemented in assembly or machine language, if desired. In any case, the language may be a compiled or interpreted language, and combined with hardware implementations. Program instructions may be executed on a single processor or on two or more processors in a distributed manner.

Examples provide a computer-implemented method for generating virtual chimera populations of multi-part organ shapes for use in an in-silico study (for example including in-silico trials or in-silico observational studies), wherein organ shapes are represented as surface or volumetric meshes, the method comprising: using a part-aware generative model to learn a latent representation of each part of a multi-part organ shape for inclusion in a virtual population and output synthesised parts of the multi-part organ shape; using a spatial composition model to align the outputted synthesised parts of the multi-part organ shape and output an anatomically meaningful example of an overall multi-part organ shape as a virtual chimera; and storing the virtual chimera in the virtual population, for use in the in-silico trial. The synthesised parts may comprise one or more meshes. The part-aware generative model may comprise an independent generator to learn a latent representation of each part of the multi-part organ shape. The independent generator may comprise a graph-convolutional variational autoencoder (gcVAE) network architecture operable to learn variability in each part of the multi-part organ shape observable across a training population. The independent generator may learn variability in each part of the multi-part organ shape observable across a training population independent to each other part. Each of the gcVAEs of the independent generator may be trained independently of each other, for example by minimising an overall loss function $L_{total}$ with respect to their constituent encoder and decoder networks' parameters respectively. The parameters of the encoder-decoder network pairs in each gcVAE may be learned iteratively, for example via the error backpropagation algorithm. The part-aware generative model may comprise a dependent generator to learn a shared latent representation of all of the parts of the multi-part organ shape. Each of the independent generator, and dependent generator, may comprise at least one encoder and at least one decoder. In examples of the dependent generator that use multiple encoder-decoder pairs, the dependent generator may further comprise at least one multiplexing layer between the encoders and the decoders, wherein the multiplexing layer is operable to provide the output of each of the encoders in use to each of the decoders in use. The dependent generator may comprise a graph-convolutional multi-channel variational autoencoder (gcmcVAE) network architecture operable to learn joint variability in shapes of each part of the multi-part organ shape observable across a training population. Joint variability may be the combined variability observed across the different organ parts, or organs of multi-part organ. The multi-part organ shape may comprise a multi-organ shape assembly. The disclosed methodologies may be nested in use to construct higher order assemblies from lower order organs - i.e. a number of singular multi-part organ shapes may be modelled, and then these are combined into an overall multi-organ assembly, up to and including a whole, entire, virtual patient. For example, a heart, a liver a kidney may all be modelled, and combined in to a Heart liver kidney assembly. The spatial composition model may further comprise at least one of an affine composition network and a non-rigid composition network. According to examples, each of: the gcVAE network; the gcmcVAE network; the affine composition network; and the non-rigid composition network may further comprise at least one residual graph convolutional down-sampling function block and at least one residual graph convolutional up-sampling function block. The at least one residual graph convolutional down-sampling function block and/or the at least one residual graph convolutional up-sampling function block may comprise at least one Chebyshev graph convolution function, at least one Exponential linear Unit function and at least one instance normalisation function. The at least one residual graph convolutional down-sampling function block may comprise a mesh pooling layer function, and/or the at least one residual graph convolutional up-sampling function block may comprise a mesh up-sampling layer function. The affine composition network may comprise at least one of: a scaling branch, a rotation branch and a translation branch. The affine composition network may further comprise a concatenation layer, and/or at least one fully connected layer (also known as dense layer or single/multi-layer perceptron). The non-rigid composition network may further comprise a feature embedding array and/or a Chebyshev convolution layer. The disclosed example methods may further comprise providing non-overlapping patient data or partially-overlapping patient data during training. The non-overlapping patient data or partially-overlapping patient data may comprises shape data extracted from a same or different patients' imaging data, wherein the imaging data may be acquired using a same or different imaging modality. This is to say, according to different examples, the overlapping nature of the data may be defined with respect of either specific patient source or of original data source (i.e. modalities) coverage. In some examples, the anatomical coverage may be a consequence of the modality of use. The different imaging modalities are capable of detecting different parameters of the patient or organ under study, such that a complete patient can be modelled by combining data from more than one modality. For example, even if modalities are looking at the same field of view, they can detect different aspects of the parts of the patient within that same field of view. By way of a specific example, a first modality may be able to image (i.e. "see") the muscular structure, and another modality may be able to image the vascular structure. Put another way, by enabling the mixing of patient imaging modalities, a more complete and accurate input training data set may be derived and used in the training of the disclosed generative system 100, and hence the end resulting synthesised variable virtual chimera are also more complete and accurate (whilst still being variable within those more accurate training derived bounds). The different imaging modalities may comprise any one or more of: magnetic resonance imaging (including cross-sectional, angiographic, or any variant of structural, functional or molecular imaging), computed tomography (including cross-sectional, angiographic, or any variant of structural, or functional imaging), ultrasound (including any variant of structural or functional), positron emission tomography, single-photon emission tomography. Each of these imaging techniques may be enhanced with appropriate endogenous or exogenous contrast media to highlight the anatomical or functional structures of interest.

Examples provide a method of training a generative shape modelling system as disclosed herein (e.g. comprising a part-aware generative model and a spatial composition model), comprising iteratively providing non-overlapping and/or partially-overlapping patient data to the generative shape modelling system for a predefined number of training epochs. The method may further comprise utilising the trained generative shape modelling system to generate (i.e. derive) a virtual chimera population. The disclosed example methods may further comprise carrying out in-silico design or testing of a medical device using the generated virtual chimera population.

Examples also provide a computer readable medium comprising instructions, which, when executed by one or more processors, causes the one or more processors to carry out any of the described methods, or parts thereof (e.g. method of training, and/or method of virtual chimera generation with the trained system).

Examples also provide an apparatus arranged or configured to carry out any of the described methods.

Examples also provide the generated output synthesised data sets, called a virtual chimera or chimera popopulation, for use in the in-silico studies as generated (i.e. derived and sysnthesied) according to the disclosed examples.

Examples also provide a method of design, or testing medical devices, comprising: training a generative shape modelling system using non-overlapping or partially overlapping patient data, (optionally testing the trained system), generating new instances of virtual chimera based on the trained generative shape modelling system, combining the new instances of virtual chimera into a virtual chimera population, designing the medical device using the virtual chimera population or testing the medical device using the virtual chimera population. Accordingly, examples of the present application provide a way of generating virtual patient populations (for, e.g., IST-based testing of medical devices against), that capture sufficient anatomical and physiological variability, representative of the myriad different potential target patient populations, to enable meaningful assessment of the respective medical device performance.

Examples according to the present disclosure may be used in any in-silico studies, including, but not limited to in-silico trials and observation studies, as used to develop medical devices such as implants meant for use in or on the patient body, but also drug development and general medical devices (including, for example, digital heath products, AI-based medical software and the like). Put another way, examples may be used with any sort of in-silico study.

The independent generator 200 and the dependent generator 300 in the disclosed system are different by design, and, as such, have different properties and advantages with respect to each other. The dependent generator can capture non-linear correlations between the input parts in the shape assembly by learning a shared latent representation across all parts. The dependent generator 300 can thus generate virtual parts (or synthetic parts) with better specificity/anatomical plausibility than the independent generator 200. As the dependent generator is trained to learn a shared latent representation that explains the variability in shape observed across all parts in the shape assembly, the shared latent representation learned in the dependent generator 300 is more constrained than the independent latent representations learned in the independent generator 200. In other words, the shared latent representation learned in the dependent generator 300 is constrained to capture the correlations that exist between the parts in the shape assembly and must be able to explain the joint variation or co-variation in shape of all parts in the shape assembly. The latent representations learned in the independent generator 200 however, do not have such a constraint, i.e. the latent representations learned for each part are completely independent of each other and are focussed only on explaining the variation in shape of their specific part. Hence, the independent generator 200 has more flexibility than the dependent generator, and, as such, can capture a greater degree of variation in shape for parts of the interest in the shape assembly. In other words, the dependent generator 300 can generate virtual chimera cohorts that are anatomically more plausible/realistic than the independent generator 200, while, the independent generator 200 can generate virtual chimera cohorts that contain more diverse shapes/capture a greater degree of variability in shape (of the individual parts and their combinations) than afforded by the dependent generator 300. The dependent generator 300 may be used instead of the independent generator 200 when the application of interest demands greater statistical fidelity. For example, in the context of conducting in-silico trials, if the trial design includes very specific inclusion and exclusion criteria to determine the virtual cohort to be used in the in-silico trial, for assessing the performance of a medical device or drug, the dependent generator 300 would be the better choice. The independent generator 200 may be used instead of the dependent generator 300 when the application of interest requires more diversity in the virtual population. For example, in the context of in-silico trials, if the aim of the trial is to explore the performance of a medical device or drug in non-standard or niche patient populations that are less prevalent/frequently observed in the real world, then the independent generator 200 would be the better choice.

While preferred embodiments of the present invention have been shown and described herein, it will be obvious to those skilled in the art that such embodiments are provided by way of example only. Numerous variations, changes, and substitutions will now occur to those skilled in the art without departing from the scope of the disclosure. It should be understood that various alternatives to the embodiments of the invention described herein may be employed in any combination in practicing the disclosure. It is intended that the following claims define the scope of the invention and that methods and structures within the scope of these claims and their equivalents be covered thereby.

**Claims**

1. A computer-implemented method for generating virtual chimera populations of multi-part organ shapes for use in an in-silico study, the method comprising:

   using a part-aware generative model to learn a latent representation of each part of a multi-part organ shape for inclusion in a virtual population and output synthesised parts of the multi-part organ shape;
   using a spatial composition model to align the outputted synthesised parts of the multi-part organ shape and output an anatomically meaningful example of an overall multi-part organ shape as a virtual chimera; and
   storing the virtual chimera in the virtual population, for use in the in-silico trial.

2. The method of claim 1, wherein the part-aware generative model comprises an independent generator to learn a latent representation of each part of the multi-part organ shape.

3. The method of claim 2, wherein the independent generator comprises a graph-convolutional variational autoencoder (gcVAE) network architecture operable to learn variability in each part of the multi-part organ shape observable across a training population.

4. The method of claim 1, wherein the part-aware generative model further comprises a dependent generator to learn a shared latent representation of all of the parts of the multi-part organ shape.

5. The method of claim 4, wherein the dependent generator comprises a graph-convolutional multi-channel variational autoencoder (gcmcVAE) network architecture operable to learn joint variability in shapes of each part of the multi-part organ shape observable across a training population.

6. The method of any of claims 1 to 5, wherein the spatial composition model further comprises at least one of an affine composition network and a non-rigid composition network.

7. The method of any of claims 3 to 6, wherein the gcVAE network, the gcmcVAE network, the affine composition network, the non-rigid composition network each further comprises at least one residual graph convolutional down-sampling function block and at least one residual graph convolutional up-sampling function block.

8. The method of claim 7, wherein the at least one residual graph convolutional down-sampling function block or the at least one residual graph convolutional up-sampling function block comprises at least one Chebyshev graph convolution function, Exponential linear Unit function or instance normalisation function.

9. The method of claim 7 or 8, wherein the at least one residual graph convolutional down-sampling function block comprises a mesh pooling layer function, and/or the at least one residual graph convolutional up-sampling function block comprises a mesh up-sampling layer function.

10. The method of any of claims 6 to 9, wherein the affine composition network comprises at least one of: a scaling branch, a rotation branch and a translation branch.

11. The method of any of claims 5 to 10, wherein the non-rigid composition network further comprises a feature embedding array or a Chebyshev convolution layer

12. The method of any preceding claim, wherein the method comprises providing non-overlapping patient data or partially-overlapping patient data during training.

13. The method of claim 12, wherein the non-overlapping patient data or partially-overlapping patient data comprises shape data extracted from a same or different patients' imaging data, wherein the imaging data is acquired using a same or different imaging modality.

14. The method of claim 13, wherein the different imaging modalities comprises magnetic resonance imaging, computed tomography, computed tomography angiography, ultrasound, positron emission tomography, single-photon emission tomography.

15. The method of any preceding claim, further comprising carrying out in-silico design, testing or regulatory approval of a medical device (including, but not limited to, implants, digital health or artificial intelligence devices), or drug using the generated virtual population.

Figure 1

Figure 8

Figure 9

Figure 2

Figure 3

**Figure 5**

Input — 501
Mesh upsampling layer — 510
Chebyshev graph convolution — 520
Instance Normalisation — 530
Exponential Linear Unit — 540
Chebyshev graph convolution — 550
Instance Normalisation — 560
Addition Layer — 570
Exponential Linear Unit — 580
output — 591
515

**Figure 4**

Input — 401
Chebyshev graph convolution — 410
Instance Normalisation — 420
Exponential Linear Unit — 430
Chebyshev graph convolution — 440
Instance Normalisation — 450
Addition Layer — 460
Exponential Linear Unit — 470
Mesh Pooling layer — 480
output — 491
415

**Figure 6**

32

(A) Input LV mesh 601A → RGCDS Block 1 610A → RGCDS Block 2 611A → RGCDS Block 3 612A → RGCDS Block 4 613A → RGCDS Block 5 614A → Fully connected Layer; Latent vector (1472x1) 615A — Sub-Network 606A

(B) Input RV mesh 601B → RGCDS Block 1 610B → RGCDS Block 2 611B → RGCDS Block 3 612B → RGCDS Block 4 613B → RGCDS Block 5 614B → Fully connected layer; Latent vector (1280x1) 615B — Sub-Network 606B

(C) Input LA mesh 601C → RGCDS Block 1 610C → RGCDS Block 2 611C → RGCDS Block 3 612C → RGCDS Block 4 613C → RGCDS Block 5 614C → Fully connected layer; Latent vector (1408x1) 615C — Sub-Network 606C

(D) Input RA mesh 601D → RGCDS Block 1 610D → RGCDS Block 2 611D → RGCDS Block 3 612D → RGCDS Block 4 613D → RGCDS Block 5 614D → Fully connected layer; Latent vector (1280x1) 615D — Sub-Network 606D

(E) Input AR mesh 601E → RGCDS Block 1 610E → RGCDS Block 2 611E → RGCDS Block 3 612E → RGCDS Block 4 613E → RGCDS Block 5 614E → Fully connected layer; Latent vector (1280x1) 615E — Sub-Network 606E

Concatenate Layer; Shared latent vector (6720x1) 616

Fully Connected Layer 1 617

Fully Connected Layer 2 618

Scaling branch: Fully connected layer 1 619 → Scaling branch: Fully connected layer 2 620 → Outputs: Scaling parameters for all five cardiac structures (5x1 vector) 621

Rotation branch: Fully connected layer 1 622 → Rotation branch: Fully connected layer 2 623 → Outputs: Rotation parameters for all five cardiac structures (20x1 vector) 624

Translation branch: Fully connected layer 1 625 → Translation branch: Fully connected layer 2 626 → Outputs: Translation parameters for all five cardiac structures (15x1 vector) 627

600

Figure 7

700

Input: coarsely aligned whole heart mesh/shape assembly (18542x3 array) 701

Encoder 706

RGCDS Block 1 — 710
RGCDS Block 2 — 711
RGCDS Block 3 — 712
RGCDS Block 4 — 713
RGCDS Block 5 — 714

Feature embedding array (24x64 array) 715

Skip Connections 717

Decoder 716

RGCUS Block 1 — 718
RGCUS Block 2 — 719
RGCUS Block 3 — 720
RGCUS Block 4 — 721
RGCUS Block 5 — 722

Chebyshev convolution layer (185428x3 array) 723

Output

Output: composed whole heart mesh/shape assembly 724

1001 → ( Start )

1010 → Generate parts using trained part-aware model

1020 → Align parts using trained spatial composition model

1030 → Store aligned parts in virtual population

1040 → ( output )

1050 → Using virtual population for design or testing devices

1000

## Figure 10

1100

1105 — HARDWARE RESOURCES

1110 — PROCESSORS

1112 — PROCESSOR

1150 — INSTRUCTIONS

1114 — PROCESSOR

1150 — INSTRUCTIONS

1140 — MEMORY/STORAGE DEVICES — 1120

INSTRUCTIONS — 1150

COMMUNICATION RESOURCES — 1130

1104 — PERIPHERAL DEVICES

1150 — INSTRUCTIONS

1108 — NETWORK

1106 — DATABASES

1150 — INSTRUCTIONS

## Figure 11

Europäisches
Patentamt

European
Patent Office

Office européen
des brevets

# EUROPEAN SEARCH REPORT

Application Number

EP 22 19 9421

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Beetz Marcel ET AL: "Generating Subpopulation-Specific Biventricular Anatomy Models Using Conditional Point Cloud Variational Autoencoders : 12th International Workshop, STACOM 2021, Held in Conjunction with MICCAI 2021, Strasbourg, France, September 27, 2021, Revised Selected Papers" In: "Advances in Visual Computing : 16th International Symposium, ISVC 2021, Virtual Event, October 4-6, 2021, Proceedings, Part II", 14 January 2022 (2022-01-14), Springer International Publishing, Cham, XP093026043, ISSN: 0302-9743 ISBN: 978-3-030-90436-4 vol. 13131, pages 75-83, DOI: 10.1007/978-3-030-93722-5_9, Retrieved from the Internet: URL:https://link.springer.com/content/pdf/10.1007/978-3-030-93722-5_9> * The whole document in particular: Abstract; Pages 75 - 78, 80 - 82; Figures 1 - 4. * | 1-15 | INV. G16H30/40 G16H50/70 G06N3/045 |
| T | HAORAN DOU ET AL: "A Generative Shape Compositional Framework: Towards Representative Populations of Virtual Heart Chimaeras", ARXIV.ORG, CORNELL UNIVERSITY LIBRARY, 201 OLIN LIBRARY CORNELL UNIVERSITY ITHACA, NY 14853, 4 October 2022 (2022-10-04), XP091333841, * The whole document, in particular: Abstract; Figures 1 - 3. * | 1-15 | |

TECHNICAL FIELDS SEARCHED (IPC)

G16H
G06N

The present search report has been drawn up for all claims

| Place of search | Date of completion of the search | Examiner |
|---|---|---|
| Munich | 22 February 2023 | Nagele, Stefan |

EPO FORM 1503 03.82 (P04C01)